(19) **Europäisches Patentamt**
**European Patent Office**
**Office européen des brevets**

(11) **EP 3 313 286 B1**

(12) **EUROPEAN PATENT SPECIFICATION**

(45) Date of publication and mention
of the grant of the patent:
**15.09.2021 Bulletin 2021/37**

(51) Int Cl.:
***A61B 5/1455*** (2006.01)     ***A61B 5/1495*** (2006.01)
***A61K 49/00*** (2006.01)

(21) Application number: **16815412.8**

(22) Date of filing: **24.06.2016**

(86) International application number:
**PCT/US2016/039334**

(87) International publication number:
**WO 2016/210311 (29.12.2016 Gazette 2016/52)**

(54) **SUBSTANCE FOR USE IN DETERMINING BIOMETRIC INDICATORS USING MULTIPLE FLUORESCENT MARKERS**

ERZEUGNIS ZUR VERWENDUNG BEI DER BESTIMMUNG BIOMETRISCHER INDIKATOREN MIT MEHREREN FLUORESZENZMARKERN

PRODUIT POUR UTILISATION DANS LA DÉTERMINATION DES INDICATEURS BIOMÉTRIQUES AU MOYEN DE PLUSIEURS MARQUEURS FLUORESCENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.06.2015 US 201562183787 P**

(43) Date of publication of application:
**02.05.2018 Bulletin 2018/18**

(73) Proprietor: **Pharmacophotonics, Inc. D/B/A Fast Biomedical**
**Carmel IN 46032 (US)**

(72) Inventors:
• **MEIER, Daniel, J.**
**Carmel, Indiana 46032 (US)**
• **SANDOVAL, Ruben, M., Jr.**
**Carmel, Indiana 46032 (US)**
• **REILLY, Erinn**
**Carmel, Indiana 46032 (US)**

(74) Representative: **Barker Brettell LLP**
**100 Hagley Road**
**Edgbaston**
**Birmingham B16 8QQ (GB)**

(56) References cited:
**EP-A1- 2 814 393      WO-A1-2015/059083**
**US-A1- 2010 268 090    US-A1- 2011 201 940**

US-A1- 2011 201 940    US-A1- 2012 276 014
US-A1- 2012 276 014    US-A1- 2014 369 936

• **EXING WANG ET AL: "A portable fiberoptic ratiometric fluorescence analyzer provides rapid point-of-care determination of glomerular filtration rate in large animals", KIDNEY INTERNATIONAL, vol. 81, no. 1, 30 August 2011 (2011-08-30), pages 112-117, XP055514970, LONDON, GB ISSN: 0085-2538, DOI: 10.1038/ki.2011.294**
• **WEIMING YU ET AL: "Rapid determination of renal filtration function using an optical ratiometric imaging approach", AJP: RENAL PHYSIOLOGY, AMERICAN PHYSIOLOGICAL SOCIETY, US, vol. 292, no. 6, 20 February 2007 (2007-02-20), pages F1873-F1880, XP002636079, ISSN: 0363-6127, DOI: 10.1152/AJPRENAL.00218.2006 [retrieved on 2007-02-01]**
• **D. K. BINDER: "In Vivo Measurement of Brain Extracellular Space Diffusion by Cortical Surface Photobleaching", JOURNAL OF NEUROSCIENCE, vol. 24, no. 37, 15 September 2004 (2004-09-15), pages 8049-8056, XP055163180, ISSN: 0270-6474, DOI: 10.1523/JNEUROSCI.2294-04.2004**

**Description**

**FIELD OF THE INVENTION**

Disclosed are compositions and methods for collecting biometric information from a mammalian subject, and preferably a human subject. More particularly, the disclosure is directed to fluorescent spectrometric methods for quantifying hematocrit and other biometric indicators of a subject by repeatedly introducing a calibrated injectate including one or more fluorescent markers into the vascular system of the subject, and monitoring the emission intensities of the fluorescent marker(s) over a period of time.

**BACKGROUND OF THE INVENTION**

Biometric indicators are valuable tools used by medical practitioners to aid in the diagnosis of a patient, and their ability to determine the proper course of medical treatment is often limited by access to rapid and accurate quantitative biometric information. Some common biometric indicators used by medical practitioners include core body temperature, blood pressure, heart and respiratory rates, blood oxygenation and hematocrit, glomerular filtration rate ("GFR"), and the like. While a medical practitioner may prefer to assess multiple biometric indicators prior to deciding on a particular treatment, the patient's condition may deteriorate faster than the indicators may be assessed. In these situations, medical practitioners are required to make decisions with limited information, potentially decreasing a patient's chance of survival. Therefore, there is a need for methods and devices capable of quickly and accurately determining one or multiple biometric indicators.

Hematocrit ("HCT"), also commonly referred to as packed cell volume, is the volumetric proportion of red blood cells to total blood volume. Hematocrit may vary based on gender and age. Typical hematocrit levels for healthy adult humans are about 45% for males and about 40% for females. HCT is commonly used in the early stages of patient care to aid in diagnosis, and abnormal HCT levels may indicate certain medical conditions. Abnormally high hematocrit levels have been associated with dengue fever, hypoxia related diseases such as COPD, dehydration, and the like, while abnormally low hematocrit levels have been associated with hemorrhaging, chronic kidney disease resulting from low erythropoietin levels, congestive heart failure and the like. There are several methods and devices currently available for determining HCT. The name "packed cell volume" is derived from the traditional method of determining HCT where centrifugal forces are applied to a heparinized sample of blood, followed by the measurement of the volumetric proportion of red blood cells to total blood volume. While this method is relatively accurate, the blood sample is often sent to a medical laboratory separate from the patient care room for analysis, which may drastically increase the sample processing time and limits its utility in time sensitive medical situations.

Invasive indirect fluorescent spectrometric methods and devices for monitoring glomerular filtration rate ("GFR") and determining the plasma volume through a single flexible optical conduit have been previously disclosed in United States Patent Application Nos. 12/425,827 and 12/946,471, and PCT Patent Application Nos. PCT/US2009/040994 and PCT/US2010/32997.

Fluorescent spectrometric systems have also been used to calculate biometric indicators. They conventionally include a light source for exciting a fluorescent marker, an optical conduit for transmitting the light source and receiving the fluorescent signal, a light detector for measuring the fluorescent signal, a means for storing the spectrometric data set, such as a digital memory storage device, a means for processing the spectrometric data set to calculate the indicator , such as a digital computation processor, and an output for the calculated indicator, such as a digital display.

GFR may be determined using a fluorescent spectrometric system by inserting the terminal end of the optical conduit into the blood vessel of an animal subject, administering a bolus injection containing a dynamic marker of a first set of fluorescent characteristics (i.e., excitation and emission wavelengths) and a steady state marker of a second set of fluorescent characteristics, fluorescently monitoring the decrease of the dynamic marker relative to the static marker over a period of time, and calculating GFR based on relative change in the markers using a series of mathematical steps. Alternatively, the volume of distribution, as defined earlier, may be determined by measuring the fluorescent signal strength of a static marker once it has reached a quasi-stable vascular distribution, and correlating the decrease in signal strength to the dilution of the bolus injection.

Conventional spectrometric methods of determining HCT assume that blood has constant optical properties during the observation period. This assumption is directly used to correlate attenuation of optical signals of multiple wavelengths to HCT independent of the length of the observation period. While conventional fluorescent injectates used to determine GFR and plasma volume are being developed for human use and have shown favorable biocompatibility, and HCT is often assessed with GFR and plasma volume in certain medical situations, they have not been used to measure HCT due to the dynamic optical properties resulting from the constantly changing concentrations of the dynamic marker used in the injectate.

Noninvasive direct spectrometric methods and devices require the use of multiple optical interfaces and optical conduits at a fixed geometry, resulting in devices that are mechanically rigid and difficult to sterilize. While fluorescent spectrometric systems are able to measure GFR and plasma volume via a single optical conduit, they are conventionally unable to measure hematocrit due to the constantly changing concentrations of the dynamic markers. Thus, there remains a clinical need to develop a sterile and accurate method of fluorescently measuring biometric indicators such as the hematocrit of a patient. EP2814393 is directed to fluorescent spectrometric methods for quantifying hematocrit and other physiological parameters of a subject by introducing a calibrated injectate comprising one or more fluorescent markers into the vascular system of the subject, and monitoring the emission intensities of the fluorescent marker(s) over a period of time. Wang et al. "A portable fiberoptic ratiometric fluorescence analyzer provides rapid point-of-care determination of glomerular filtration rate in large animals", Kidney International (2012) discusses a rapid, point of care determination of glomerular filtration rate.

## SUMMARY OF THE INVENTION

The present disclosure relates to methods of measurement of biometric indicators in a mammalian subject. Representative biometric indicators of interest include hematocrit, plasma volume, volume of distribution, and glomerular filtration rate. Biometric indicators such as hematocrit, glomerular filtration rate and plasma volume may be measured by administering an injectate with a dynamic fluorescent marker (i.e., a dynamic molecule labeled with a first fluorescent tag) and a static fluorescent marker (i.e., a static molecule labeled with a second fluorescent tag, wherein the first and second tags have distinct (non-overlapping) fluorescent characteristics that enables them to be separately detected) into the vasculature of the mammalian subject. Biometric indicators such as hematocrit and plasma volume (but not GFR) may also be determined by administering an injectate containing a single static marker labeled with two fluorescent tags, into the vascular system of the subject. The markers of the present invention may also be described herein in terms of a fluorescent tag being "conjugated" to or "associated with" a static or dynamic marker. This terminology is not meant to imply any particular chemical means by which the dynamic or static molecule is "labeled" with the tag. The methods entail measuring the emission intensities of the fluorescent tags over a period of time with one or more measurements, depending on the indicator that is being determined. For example, HCT and PV may be measured via a single measurement (as the term is used herein) whereas GFR may be determined on the basis of three measurements conducted at predetermined times after administration of the injectate.

The invention is set out in the independent claim. Preferred embodiments are set out in the dependent claims.

Disclosed herein is a method for measuring a biometric indicator of a mammalian subject, comprising

(a) calibrating an injectate to obtain a calibration identification of the injectate that contains parameters of the injectate, wherein the injectate includes:

(i) a first fluorescent tag having a first excitation wavelength and a first emission wavelength;
(ii) a second fluorescent tag having a second excitation wavelength and a second emission wavelength, wherein both first and second fluorescent tags are conjugated to a static molecule, or wherein the first fluorescent tag is conjugated to the static molecule and the second fluorescent tag is conjugated to a dynamic molecule; and
(iii) an injectate carrier;

(b) inputting the parameters of the calibration identification of the injectate to a fluorescent detector to calibrate the fluorescent detector;
(c) determining existing level of fluorescence in the mammalian subject;
(d) introducing the injectate into vascular system of the mammalian subject;
(e) exciting the first fluorescent tag with a first excitation wavelength and exciting the second fluorescent marker with a second excitation wavelength at an optical interface of an optical probe and the vascular system, or in a sample taken from the vascular system of the mammalian subject;
(f) measuring the first emission intensity of the first emission wavelength of the first fluorescent tag and measuring the second intensity of the second emission wavelength of the second fluorescent tag at the optical interface of the optical probe and the vascular system, or in the sample taken from the vascular system of the mammalian subject, using the calibrated fluorescent detector, to obtain a spectrometric data set, which includes a first emission fluorescent intensity curve from the first fluorescent tag and a second emission fluorescent intensity curve from the second fluorescent tag;
optionally (g) periodically repeating steps (e) through (f) or steps (c) through (f); and
(h) calculating the biometric indicator of the mammalian subject as a function of the first and second emission intensities measured in (f) and relative to the existing level of fluorescence measured in (c).

In one aspect, the disclosure is directed to a method for measuring plasma volume (PV) of a mammalian subject. The method may include:

(a) calibrating an injectate to obtain a calibration identification of the injectate that contains parameters of the injectate, wherein the injectate includes:

(i) a first fluorescent tag having a first excitation wavelength and a first emission wavelength;
(ii) a second fluorescent tag having a second excitation wavelength and a second emission wavelength, wherein both first and second fluorescent tags are conjugated to a static molecule, or wherein the first fluorescent tag is conjugated to the static molecule and the second fluorescent tag is conjugated to a dynamic molecule; and
(iii) an injectate carrier;

(b) inputting the parameters of the calibration identification of the injectate to a fluorescent detector to calibrate the fluorescent detector;
(c) determining existing level of fluorescence in the mammalian subject;
(d) introducing the injectate into vascular system of the mammalian subject;
(e) exciting the first fluorescent tag with a first excitation wavelength and exciting the second fluorescent marker with a second excitation wavelength at an optical interface of an optical probe and the vascular system, or in a sample taken from the vascular system of the mammalian subject;
(f) measuring the first emission intensity of the first emission wavelength of the first fluorescent tag and measuring the second intensity of the second emission wavelength of the second fluorescent tag at the optical interface of the optical probe and the vascular system, or in the sample taken from the vascular system of the mammalian subject, using the calibrated fluorescent detector, to obtain a spectrometric data set, which includes a first emission fluorescent intensity curve from the first fluorescent tag and a second emission fluorescent intensity curve from the second fluorescent tag; and
optionally (g) periodically repeating steps (e) through (f) or steps (c) through (f); and
(h) calculating the plasma volume of the mammalian subject as a function of the first and second emission intensities measured in (f) and relative to the existing level of fluorescence measured in (c).

Thus, plasma volume may be determined on the basis of a single measurement subsequent to the introduction of the injectate, typically about 10-15 minutes after the first introduction thereof. A plasma sample can be excited with an appropriate light source to conduct step (e). Changes in plasma volume may be easily monitored by taking additional e.g., two, measurements, timing of which is not critical, and which typically may be about 60 minutes and 120 minutes after the introduction of the injectate. Step c may be conducted any time prior to introduction of the injectate, typically about 30 minutes prior thereto.

As used in the present invention with respect to all aspects thereof, the term "measurement" or "measuring" differs depending upon whether it is conducted using a probe or a blood (or plasma) sample obtained from the mammalian subject. Thus, in embodiments of the invention that are practiced using a probe, the "measuring" or "measurement(s)" is, in reality and as a person skilled in the art would readily appreciate, a group of readings each of which contains a rate and a magnitude. The probe monitors fluorescent emission intensities in real time and contains a richer data set relative to measurements obtained using a blood sample. Thus, by way of illustration, a measurement conducted at 10 minutes post-administration of the injectate may be thought of as a 10 minute data set (in the case of a probe), or a single reading (in the case of a blood sample), and a reading 60 minutes after administration of the injectate (e.g., such as might be taken in calculating GFR, as described herein) which would be a post-equilibrium reading, would be considered as a 60-minute data set when obtained using a probe, or, once again, a single reading at about 60 minutes if obtained from a sample.

Another aspect of the disclosure is directed to a method for measuring glomerular filtration rate (GFR) of a mammalian subject. The method may include:

(a) calibrating an injectate to obtain a calibration identification of the injectate that contains parameters of the injectate, wherein the injectate includes:

(i) a first fluorescent tag having a first excitation wavelength and a first emission wavelength;
(ii) a second fluorescent tag having a second excitation wavelength and a second emission wavelength, wherein the first fluorescent tag is conjugated to a static molecule ("a static marker") and the second fluorescent tag is conjugated to a dynamic molecule ("a dynamic marker"); and
(iii) an injectate carrier;

(b) inputting the parameters of the calibration identification of the injectate to a fluorescent detector to calibrate the fluorescent detector;

(c) determining existing level of fluorescence in the mammalian subject;

(d) introducing the injectate into vascular system of the mammalian subject;

(e) exciting the first fluorescent tag with a first excitation wavelength and exciting the second fluorescent marker with a second excitation wavelength at an optical interface of an optical probe and the vascular system, or a sample taken from the vascular system;

(f) measuring the first emission intensity of the first emission wavelength of the first fluorescent tag and measuring the second intensity of the second emission wavelength of the second fluorescent tag at the optical interface of the optical probe and the vascular system, or in the sample taken from the vascular system of the mammalian subject, using the calibrated fluorescent detector, to obtain a spectrometric data set, which includes a first emission fluorescent intensity curve from the first fluorescent tag and a second emission fluorescent intensity curve from the second fluorescent tag, wherein the second fluorescent intensity curve further comprises peak fluorescent intensity of the second fluorescent tag conjugated to the dynamic marker which is extrapolated from amount of the second fluorescent tag contained in the injectate, and plasma volume;

(g) repeating steps (e) through (f) at least twice or repeating steps (c) through (f) at least twice, wherein a first repetition is conducted at a time coinciding with or after equilibrium of the dynamic marker in plasma and interstitial fluid of the mammalian subject has been achieved; and

(h) calculating the GFR of the mammalian subject as a function of the first and second emission intensities measured in (f) and (g) and relative to the existing level of fluorescence of (c).

Thus, determination of GFR entails use of two fluorescent tags one of which is conjugated to a static marker and the other conjugated to a dynamic marker, an extrapolation of peak fluorescent intensity of the tag conjugated to the dynamic marker (at To, which cannot be measured directly), and at least 3 measurements of fluorescence intensity (e.g., typically about 10-15 minutes, about 30-60 minutes and then about 120 minutes after the introduction of the injectate into the vasculature. Following the initial dose of the injectate, the concentrations of the markers are substantially higher in plasma relative to interstitial (e.g., non-circulatory) fluid. The dynamic marker diffuses or leaks through the capillaries of the vasculature into interstitial fluid relatively quickly compared to the static marker, and ultimately reaches a point of equilibrium, wherein concentration of the dynamic marker in the plasma is substantially equal to concentration of the dynamic marker in the interstitial fluid. In the methods of determining GFR, the second measurement is conducted only after this equilibrium is achieved which typically occurs in about 30-60 minutes (and for subjects with slower circulation may even be longer (e.g., about 70, 80 or 90 minutes)), and the third measurement is taken at a predetermined time thereafter, e.g., about 120 minutes after administration of the injectate. In some embodiments, the second and third measurements are taken following "follow on" doses of the injectate, in which case step g entails repeating steps c through f at least twice. Thus, in these embodiments, which are referred to herein as "multi-dosing" or "follow on dosing", another baseline measurement (step c) is conducted prior to each and every successive dose of the injectate. In these embodiments, the first follow-on dose is administered once equilibrium of the dynamic markers has been achieved.

Further aspects of the present disclosure are directed to a method for measuring HCT. One such aspect entails use of two distinct fluorescent tags, which are associated with (e.g., conjugated to) different molecules, namely a static molecule and a dynamic molecule. In such aspect, the method for measuring hematocrit (HCT) of a mammalian subject may include:

(a) calibrating an injectate to obtain a calibration identification of the injectate that contains parameters of the injectate, wherein the injectate includes:

(i) a first fluorescent tag having a first excitation wavelength and a first emission wavelength;

(ii) a second fluorescent tag having a second excitation wavelength and a second emission wavelength, wherein the first fluorescent tag is conjugated to a static molecule ("a static marker") and the second fluorescent tag is conjugated to a dynamic molecule ("a dynamic marker"); and

(iii) an injectate carrier;

(b) inputting the parameters of the calibration identification of the injectate to a fluorescent detector to calibrate the fluorescent detector;

(c) determining existing level of fluorescence in the mammalian subject;

(d) introducing the injectate into vascular system of the mammalian subject;

(e) exciting the first fluorescent tag with a first excitation wavelength and exciting the second fluorescent tag with a second excitation wavelength at an optical interface of an optical probe and the vascular system, or a sample taken from the vascular system;

(f) measuring the first emission intensity of the first emission wavelength of the first fluorescent tag and measuring the second intensity of the second emission wavelength of the second fluorescent tag at the optical interface of the optical probe and the vascular system, using the calibrated fluorescent detector, to obtain a spectrometric data set, which includes a first emission fluorescent intensity curve from the first fluorescent tag and a second emission fluorescent intensity curve from the second fluorescent tag, wherein the second fluorescent intensity curve further comprises peak fluorescent intensity of the second fluorescent tag conjugated to the dynamic molecule which is extrapolated from amount of the second fluorescent tag contained in the injectate, and plasma volume;
optionally (g) periodically repeating steps (e) through (f) or steps (c) through (f); and
(h) calculating the HCT of the mammalian subject as a function of a raw ratio of the first and second peak emission intensities measured in (f), and which is relative to the existing level of fluorescence measured in (c) and a species-specific HCT curve obtained prior to (h).

In this aspect of the disclosure, HCT can be determined using two distinct fluorescent tags, one being conjugated to a static molecule and another being conjugated to a dynamic molecule. Thus, HCT may be determined on the basis of a single dataset subsequent to the introduction of the injectate, typically about 10-15 minutes after the introduction thereof. The dataset may contain both rate of change and intensity. Step c may be conducted any time prior to introduction of the injectate, typically about 30 minutes prior thereto. HCT can be measured using a probe once that is at the "extrapolated" To point using the early phase decay data set (which since it is taken about 10-15 minutes after introduction of the injectate, occurs before equilibrium).

Alternatively, HCT may be measured using two distinct fluorescent tags, each of which is associated with (e.g., conjugated to) the same molecule, namely a static molecule. In this aspect of the disclosure, the method for measuring hematocrit (HCT) of a mammalian subject may include:

(a) calibrating an injectate to obtain a calibration identification of the injectate that contains parameters of the injectate, wherein the injectate includes:

(i) a first fluorescent tag having a first excitation wavelength and a first emission wavelength;
(ii) a second fluorescent tag having a second excitation wavelength and a second emission wavelength, wherein the first fluorescent tag and the second fluorescent tag are each conjugated to a static molecule; and
(iii) an injectate carrier;

(b) inputting the parameters of the calibration identification of the injectate to a fluorescent detector to calibrate the fluorescent detector;
(c) determining existing level of fluorescence in the mammalian subject;
(d) introducing the injectate into vascular system of the mammalian subject;
(e) exciting the first fluorescent tag with a first excitation wavelength and exciting the second fluorescent tag with a second excitation wavelength at an optical interface of an optical probe and the vascular system, or a sample taken from the vascular system;
(f) measuring the first emission intensity of the first emission wavelength of the first fluorescent tag and measuring the second intensity of the second emission wavelength of the second fluorescent tag at the optical interface of the optical probe and the vascular system, or a sample taken from the vascular system, using the calibrated fluorescent detector, to obtain a spectrometric data set, which includes a first emission fluorescent intensity curve from the first fluorescent tag and a second emission fluorescent intensity curve from the second fluorescent tag;
optionally (g) periodically repeating steps (e) through (f) or steps (c) through (f); and
(h) calculating the HCT of the mammalian subject as a function of a raw ratio of the first and second peak emission intensities measured in (f), and which is relative to the existing level of fluorescence measured in (c) and a species-specific HCT curve obtained prior to (h).

In this aspect, probes or a blood sample may be used. Also in this aspect, since it is unnecessary to extrapolate to $T_0$, the raw ratio is the HCT (relative to the existing fluorescence (c) and the species-specific HCT curve).

For purposes of the present invention, an "extrapolated $T_0$", which is a curve fitting decay vs time data to find what it would be at $T_0$ (which is conducted in the course of determining GFR and HCT using the static marker and the dynamic marker), and a direct calculation at $T_0$ using plasma volume data derived from a blood sample version of the method, must be differentiated from each other. In the case of probe data, there may be hundreds of measurements taken over a few minutes for each "data set", thus enabling an extrapolation of the $T_0$ value. In the case of blood samples, the $T_0$ value can be measured directly based on a known PV, because the two values are related to each other. Thus, the two known values may be used to derive a single unknown. VFI concentration and PV thus allow a calculated $T_0$ concentration of the dynamic marker (i.e., the fluorescent tag associated with the dynamic molecule). This method is more accurate than extrapolation. In the case of blood samples, it may be desirable or even necessary to obtain many (e.g., 6 or more) blood samples to extrapolate $T_0$ values with only a single marker system, and they would need to be taken within the first 30 minutes. Thus, for purposes of the present invention, methods for calculating HCT using two markers is simpler and faster than methods using a single static molecule associated with two distinct fluorescent tags (also referred to herein as a "single marker"), at least with respect to practicing same using blood samples.

In aspects for determining the HCT using probes, PV can be calculated based on HCT value.

At least two doses of the injectate (also referred to as follow-on doses) are administered to the mammalian subject. In such methods, multiple doses of the markers are administered over a test period. Using this technique it can be shown that as the total concentration of markers increases in both the interstitial and bladder spaces during a follow on test, the rate of decay remains relative to that total dose minus the amount cleared. Even though the interstitial concentration of the markers also grows, the rate of leakage remains the same, as the change in concentration is the only factor affecting the leakage rate.

The invention may be used in combination with known methods and devices to achieve simultaneous measurement of blood volume, plasma volume, volume of distribution, glomerular filtration rate (GFR) and hematocrit from a single injection of the injectate.

Other features and advantages of the present invention will be apparent from the following description.

## BRIEF DESCRIPTION OF THE DRAWINGS

FIG. 1 is an example of the results of a step dose blood test set.

FIG. 2 is a plot of each VFI component (intercept forced to zero), using the average signal level and amount of each component at each dose step.

FIG. 3 is a plot of fluorescence intensity level vs. HCT.

FIG. 4 is plot of the HCT data of FIG. 3 taking the ratio of the signal levels of Component 1 to Component 2, and plotting that ratio versus the HCT calculated at each stage.

FIG. 5 is an example of a spectrometric data set obtained from administering and fluorescently monitoring of the vascular distribution of an injectate of the present invention.

FIG. 6 is an example of a calibration curve of the fluorescence intensity signal level vs. material amount;

FIG. 7 is an example of a calibration curve of the fluorescence intensity signal level vs. HCT.

FIG. 8 is an example of a calibration curve of the raw ratio (concentration ratio of the dynamic and static markers at $T_0$) of the fluorescent markers vs. HCT.

FIG. 9 is an example of a calibration curve of fluorescent intensity signal level vs. HCT using a single static marker with two fluorescent tags.

Fig. 10 illustrates a spectrometric data set obtained from a administering and fluorescently monitoring of the vascular distribution of an injectate multiple times over a test period as would be characterized by a normal mGFR.

Fig. 11 illustrates a spectrometric data set obtained from a administering and fluorescently monitoring of the vascular distribution of an injectate multiple times over a test period as would be characterized by an impaired mGFR.

## DETAILED DESCRIPTION OF THE INVENTION

For the purposes of promoting an understanding of the principles of the invention, reference will be made to the embodiments illustrated in the drawings and specific language will be used to describe the same. It will nevertheless be understood that no limitation of the scope of the disclosure, with such alterations and further modifications in the illustrated device and such further applications of the principles of the technology as illustrated therein being contemplated as would normally occur to one skilled in the art to which the technology relates.

The methods of the present disclosure may be practiced with an injectate, also referred to herein as an "visible fluorescent injectable (VFI)", a device or group of devices capable of measuring fluorescent intensity, and a group of mathematical algorithms capable of determining different biometric indicators based upon the data collected from the VFI and the device(s). As disclosed herein, the VFI may, in some embodiments, include two dextran molecules, of differing molecular weights, conjugated to 2 fluorescently distinct tags, e.g., dyes. Thus, in some embodiments, a first high molecular weight dextran molecule may be conjugated to a fluorescent "red dye", and another, low molecular weight dextran may be conjugated with fluorescent "green dye." The "device" may be a probe-based instrument such as a ratiometric fluorescent device (RFD), which is designed to work in concert with a probe, such as an invasive probe, e.g., one designed to insert into the vein of a mammalian subject, as well as with non-invasive probes, e.g., oral probes that are capable of measuring fluorescent intensity through the skin of the mouth. Other devices that may be used in the practice of the present invention include blood sample reading devices, such as clinical lab-based instruments that use blood samples spun down to yield plasma, and bedside instruments that are capable of reading fluorescence through whole blood, in accordance with the present invention, require a "correction" in order for accurate determination of HCT. Each biometric indicator that can be measured in accordance with the present invention requires different parts of the data set collected by the devices, in different mathematical equations used to obtain the necessary measurements. Illustrations of such equations and measurements are illustrated in the working examples.

Plasma volume (PV) may be determined using a single static marker which has two fluorescently distinct tags conjugated thereto. PV may be derived by optionally taking a blank (pre-dose) sample to measure residual or background or existing fluorescence, followed by a measurement of fluorescent intensity of the tags after "distribution" of the marker occurs, e.g., usually in about 10-15 minutes following administration of the injectate. As used herein, the term distribution refers to a time when the marker (or markers) has mixed thoroughly into the blood plasma. The data set is then used to calculate PV by measuring concentration of the large marker in the blood plasma (VFI (dose concentration) ones divided by the measured concentration). This value directly measures PV. Optionally, additional samples can be taken over time to monitor changes in PV. This monitoring allows a clinician to perform interventions and thus monitor how PV has changed. In turn, blood volume can be derived by adding back to the total volume, the amount of HCT contained in the subject. The HCT total volume plus plasma volume is equal to blood volume.

GFR can also be determined in several ways using the present invention. In some embodiments, blood samples are taken at different time points. Persons skilled in the art may optionally take a blank (pre-dose value) measurement, which is used to determine any residual/background/existing level of fluorescence. This measurement is especially advantageous in those embodiments in which repeat or follow-on dosing of the VFI is conducted. Data are then collected from samples at about 3 time points, e.g., 10-15 minutes, about 60 minutes and about 120 minutes. A calculation at $T_0$, which is done by using the PV value of the large marker and dividing by the concentration of the small marker in the VFI, enables persons skilled in the art to derive the rapid phase of the clearance between $T_0$ and the time point at 10-15 minutes. Once equilibrium has been established (as the term is used herein), the data sets obtained from the measurements at about 60 and at about 120 minutes allows for the determination of the slow phase clearance. Then, one may mathematically derive an area under the curve (AUC) of the total clearance, yielding the GFR. Alternatively, GFR may be determined using a probe-based system, which may entail first generating an HCT value (as described below) and then collecting data sets before the VFI is injected (pre-dose), another data set prior to equilibrium (e.g., before and up to about 10-15 minutes), and then at post-equilibrium (about 60-120 minutes). These data sets are then used in the same way as described above in the context of blood samples. Whole blood samples can be used without spinning down to isolate plasma, but in these embodiments, persons skilled in the art would need to know the HCT value (which can be measured in accordance with standard techniques, such as by capillary centrifuge). Subsequent data sets can be taken, e.g., at 120 and 180 minutes, etc., and which can be used to update the value of the slow clearance phase, and a new AUC curve derived to show changes in GFR over time.

HCT may be used for correction of the probe-based system with a RFD device. These devices are capable of continuous readings of a fluorescent signal in whole blood that is flowing within the body. The present invention utilizes data sets taken at certain times to produce the HCT. Thus, in the case of probes, a data set taken within the first 10-15 minutes can be used to extrapolate back to the intensity that would have been determined at $T_0$ (time 0, which as used herein, refers to a point that cannot be measured directly but can be mathematically derived by curve fitting equations to yield an intensity equivalent to the starting concentration of the fluorescent tags in the VFI). The raw ratio of the two intensities (e.g., green versus red tags or dyes) can then be used to determine the HCT value, which can be done by using a previously-calibrated HCT curve of the mammalian subject being tested.

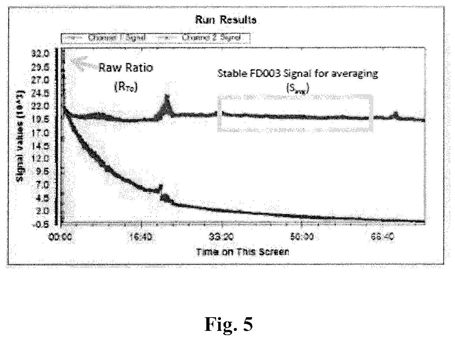

The disclosure generally relates to compositions and methods for the measurement of biometric indicators in a mammalian subject. The mammalian subject may be a human. The biometric indicators of interest include, but are not limited to, hematocrit, blood volume, plasma volume, volume of distribution, and glomerular filtration rate (GFR). The invention may be especially applicable to subjects having rapid blood loss whose hematocrits are unknown and subjects with unstable hematocrits. As defined in the present application, the term "plasma volume" refers to the total amount of plasma contained in the vasculature of a subject, while the term "circulating plasma volume" refers to the amount of flowing plasma contained in the vasculature of the subject. Although the measurements for "plasma volume" and "circulating plasma volume" are similar and related, they are not the same. Hematocrit may be determined by analyzing a spectrometric data set, as shown in FIG. 5, obtained from the administration and fluorescent monitoring of the vascular distribution of an injectate for a period of time that includes the peak vascular distribution of the markers at $T_0$. A calibrated spectrometric analyzer of the present disclosure may be used to determine HCT from the spectrometric data set. One advantage of an aspect of the invention is the ability to utilize dynamic and static markers to determine HCT in a subject.

A spectrometric data set as used in the present application means a data set resulting from the administration and fluorescent monitoring of the vascular distribution of an injectate containing two or more fluorescent markers of distinct fluorescent characteristics, where one of the fluorescent markers is a dynamic marker and one of the fluorescent markers is a static marker, or wherein both fluorescent markers are associated with a static molecule, for a period of time that includes the peak vascular distribution of the fluorescent markers.

A calibrated spectrometric analyzer of the disclosure ("Calibrated Spectrometric Analyzer") includes an input for a spectrometric data set, an input for calibration identification, a computational engine for calculating hematocrit, and an output for reporting a calculated hematocrit. The calibration identification may be set with factory predicted average injectate parameters during manufacturing and stored in a computationally accessible location, it may be updated indirectly via a change in software or hardware, or may be updated directly by uploading injectate specific parameters. Injectate specific parameters may be inputted through the use of a manual device, such as a keypad or touch screen, through the use of a semi-automated device, such as a barcode scanner, or through the use of an indirect automated process, such as by the use of a wireless software update.

Injectates

The injectate of the present invention includes a first fluorescent marker, a second fluorescent marker, and an injectate carrier. Each fluorescent marker has its own distinct fluorescent characteristics, i.e. distinct excitation wavelengths and emission wavelengths. The first fluorescent marker has a first excitation wavelength and a first emission wavelength. The second fluorescent marker has a second excitation wavelength and a second emission wavelength. A fluorescent marker is any molecule containing a fluorophore (also defined to herein as a tag such as a dye) which causes the molecule to be fluorescent. Many known fluorescent dyes can serve as fluorescent markers of the present invention, such as but not limited to rhodamine dyes or its derivatives (e.g., 2-sulfhydroRhodamine (2SHR) and Texas Red®), fluorescein or its derivatives (e.g. fluorescein isothiocyante (FITC)), coumarin and cyanine, all of which have distinct excitation and emission wavelengths from each other. The fluorescent tag may be associated with, for example via conjugation, another macromolecule (a labeled macromolecule) to provide an intended molecular weight for the fluorescent dye. Examples of macromolecules include but are not limited to polymers, proteins, dextrans, celluloses, carbohydrates and nucleic acids. The macromolecules can be naturally occurring compounds, or synthetic compounds. Methods for conjugating macromolecules with fluorescent dyes are well known in the art.

The first fluorescent marker is a dynamic molecule labeled with a first fluorescent tag, and the second fluorescent marker is a static molecule labeled with a second fluorescent tag.

A "dynamic molecule" is a molecule of sufficiently low molecular mass to permeate the blood vessel walls or the vasculature of a subject. Dynamic molecules are known in the art to have a molecular mass less than 50 kDa, and more typically have a molecular mass less than 20 kDa.

A "static molecule" is a molecule of sufficiently high molecular mass to significantly limit its blood vessel wall permeability. Static markers may reach a quasi-stable vascular concentration for a period of time, although such markers may ultimately be cleared from the vasculature. Static markers are known in the art to have a molecular mass greater than 50 kDa, and more typically have a molecular mass greater than 200 kDa. Such markers can remain in the vasculature for a time period of between about 1 or 2 hours, to 12 hours or longer, depending on the molecular mass of the marker as well as other factors.

Thus, by way of example, a first fluorescent marker may include a dynamic molecule such as a 5-7 kDa dextran, conjugated to a fluorescein dye, and the second fluorescent marker may include a static molecule such as a 150 kDa dextran conjugated to 2SHR.

In another embodiment, the injectate may include a static marker having two fluorescent tags attached thereto and an injectate carrier. Each fluorescent tag has its own distinct fluorescent characteristics, i.e. distinct excitation wavelengths and emission wavelengths. An example of such a static marker is a macromolecule, such as dextran with molecular mass greater than 50 kDa, labeled with (e.g., conjugated with) two different fluorescent dyes, such as Texas Red® and fluorescein or a derivative thereof.

The fluorescent markers are not metabolized within the subject during the period of time of measuring the biometric indicators. What is meant by "not metabolized within the subject" in the present application is that the marker has a half-life ($T_{1/2}$) of 4 hours or greater in the vascular system of the subject.

In the present invention, the two injectates can be used substantially interchangeably. That is, with the exception of measuring GFR, it is not important whether the injectate has two separate fluorescent markers providing two distinct fluorescent characteristics, or whether the injectate has only one marker having two fluorescent tags providing two distinct fluorescent characteristics. What is important is that the injectate provides two distinct fluorescent emission signals in order to allow the measurement of the biometric indicators as described in the present application. Thus, when reference is made to using an injectate having two fluorescent markers in the present application, this is also intended to include and refer to an injectate having only one marker but with two fluorescent tags on the molecule. The subsequent steps leading to the measuring of the hematocrit and other biometric indicators are otherwise identical. However, since the injectate including only one molecule uses a static marker without a dynamic marker, the injectate can be used to measure the hematocrit and other biometric indicators, but not GFR which requires at least two markers.

The term "injectate carrier" as used in the present application means a biologically acceptable fluid capable of solubilizing and delivering the fluorescent markers to aid in the delivery and biocompatibility of the fluorescent markers. Examples of suitable carriers include but are not limited to buffers, saline (e.g., physiologically buffered saline) and the like.

The injectate may be introduced into the vascular system via bolus injection or by infusion.

Calibration Identification and Calibration Identifier

The injectate of the disclosure is calibrated to provide a calibration identification that contains parameters of the injectate.

The term "calibration identification" as used in the disclosure means a collection of fluorescent injectate parameters that are used in the calculation of the biometric parameter such as HCT from a spectrometric data set. The parameters may include the Visible Fluorescence Injectate (VFI) lot number and calibrated fluorescent intensity of each fluorescent marker or each fluorescent tag on the same marker.

A calibration identification can be represented as a calibration identifier represented by a series of numbers or signals. In an embodiment, the series of numbers or signals may be an optical machine-readable representation of data, such as but not limited to bar codes. Algorithms to convert the calibration identifier to a bar code calibration identifier are well known to those in the art.

The calibration identification may be set with factory predicted average injectate parameters during manufacturing, and is stored in a computationally accessible location. It may be updated indirectly via a change in software or hardware, or may be updated directly by uploading injectate specific parameters.

Injectate-specific parameters contained in the calibration identification may be inputted into another device, such as a fluorescent detector or a spectrometric analyzer, through the use of a manual device, such as a keypad or touch screen, through the use of a semi-automated device, such as a barcode scanner, or through the use of an indirect automated process, such as a wireless software update.

The reference standard fluorescent intensities used to generate the calibration curves which in turn are used to calculate the biometric parameters may be represented in the calibration identifier as set value 1000 with an immediately following letter designation for each fluorescent marker of different fluorescent wavelength immediately following (i.e., 1000a; 1000b). Fluorescent intensity variance from the reference standard for each fluorescent marker may be represented in the calibration identifier as a representative equivalent increase or decrease to the set value of 1000.

A sample calibration identifier is shown below:

LOTIOIAI034B0975

Information contained:

VFI Lot No.: 101
Fluorescent Marker 1 (A) intensity from calibration: 1034
Fluorescent Marker 2 (B) intensity from calibration: 0975

Calibrated Injectate

A calibrated injectate of the disclosure ("Calibrated Injectate") may include a first fluorescent marker or fluorescent tag having a first hematocrit-dependent fluorescent attenuation coefficient, a second fluorescent marker or fluorescent tag having a second hematocrit-dependent fluorescent attenuation coefficient, an injectate carrier, and a calibration identification. The calibration identification may be provided separately from the Calibrated Injectate, may be provided with the Calibrated Injectate, or may be provided as a Calibration Identification. A Calibrated Injectate may be used to further improve the accuracy and precision of a Calibrated Spectrometric Analyzer by correcting for the optical batch variance resulting from the multiple manufacturing steps.

A calibration method of the disclosure used to produce a Calibrated Injectate may include a set of fluorescent intensity standards for each fluorescent marker or fluorescent tag, a set preparation procedure for creating working standard solutions and calibration solution for calibrating a fluorescence detector, and a fluorescence detector used to read the fluorescent intensity of each fluorescent marker in calibrations solution and injectate. From fluorescent marker standard solutions the set procedure is followed to create a working standard solution and a calibration solution. The calibration solution is used in the same fluorescent intensity range for each marker as the injectate. The calibration solution is used to set the parameters of the fluorescence detector. Then using the same set procedure, a test solution is made using the injectate to be calibrated. Using the calibrated fluorescence detector, the injectate test solution for the calibration identification for a Calibrated Injectate is generated.

A Calibrated Spectrometric Analyzer

A calibrated spectrometric analyzer of the disclosure ("Calibrated Spectrometric Analyzer") includes an input for a spectrometric data set, an input for a calibration identifier, a computational engine for calculating hematocrit, and an output for reporting a calculated hematocrit.

Method for Determining Hematocrit

Hematocrit may be determined by analyzing a spectrometric data set obtained from administering and fluorescently monitoring of the vascular distribution of an injectate containing two or more fluorescent markers of different fluorescent wavelengths, where at least one of the fluorescent markers is a dynamic marker, for a period of time that includes the peak vascular distribution of the markers. Alternatively, the injectate may contain only one static marker having two fluorescent tags on the marker. A calibrated spectrometric analyzer of the present disclosure may be used to determine HCT from a spectrometric data set. One advantage of the invention is its ability to utilize a combination of dynamic static markers, or a static marker (associated with two distinct fluorescent tags) to determine HCT in an animal subject.

The term "time zero" or "$T_0$", as used herein, is the point in time that the injectate is introduced into the vasculature of the mammalian subject. It may also coincide with the moment in a spectrometric data set that is characterized by the peak fluorescent signal intensity of intravenously injected fluorescent markers (and thus the point of initial analysis for the math). Thus, $T_0$ is used to signify the start of the biometric parameter fluorescent signal analysis. The term "raw ratio" as used herein may be defined as the ratio of fluorescent signal intensities of the two fluorescent tags at $T_0$, i.e. the ratio of the dynamic marker ("small marker", indicating a smaller molecular weight, or "green marker", indicating a fluorescent tag emitting in the green spectrum) to the static marker ("larger marker", indicating a larger molecular weight, or a "red marker", indicating a fluorescent tag emitting in the red spectrum). An important aspect of the present technology is the use of the raw ratio to determine HCT in an optically dynamic environment.

It has been found that up to one-half of the small marker is filtered from the blood stream after only about 15 minutes following the initial bolus infusion of a dynamic marker and a static marker, which in some embodiments totals about 3ml. Accordingly, following the procedure of the present invention, the concentration of the dynamic marker at $T_0$ can be accurately predicted using, for instance, a spectrometric analyzer to measure the concentration of the static marker at 10 to 15 minute intervals as described herein. This is a significant advance since it permits the use of periodic biometric sampling, e.g., sampling the vasculature every 10 to 60 minutes or 3 times over 2 hours (which may be done to calculate plasma volume and GFR), as contrasted to a continuous sampling procedure. Thus the total test time can be shortened to about 1 to 2 hours in duration from about 6 hours required by the current methods. The "sampling" may be conducted in accordance with techniques known in the art, e.g., via blood samples and use of invasive (e.g., venous) or non-invasive (e.g., oral) probes.

The raw ratio may be used, in turn, to calculate the hematocrit observed at the optical interface of an optical probe, referred to herein as the apparent HCT. The apparent hematocrit obtained from invasive (e.g., venous) probes may be different from a subject's true HCT. This may be attributed to fluid dynamic anomalies occurring near an optical interface inserted in a flowing system. True HCT may be calculated from apparent HCT by applying a correction factor. A correction factor may be in the range of 1 to 10 percent of apparent HCT, and more specifically in the range of 4-5 percent of HCT. A typical calculation of the correction factor is shown in the Examples herein. Thus, a correction function is not necessary when the invention is carried out with non-invasive probes such as oral probes.

A method for determining a species specific HCT curve may utilize the following components: a calibrated fluorescence detector, a Calibrated Injectate, and a test volume of species specific blood. A procedure may be performed to maintain a constant total test volume and constant concentration of Calibrated Injectate in the test volume while altering the HCT in the test volume. A calibrated fluorescence detector is set up and configured to read the fluorescence intensities of the test volume throughout the procedure. A test volume is prepared, with a known HCT ($H_{calib}$), as determined by conventional methods, and a measured total volume ($V_t$). A known volume of Calibrated Injectate is added to the test volume. A separate volume is created from normal saline and Calibrated Injectate, with an equivalent concentration of Calibrated Injectate added to the test volume. This solution is used to replace removed volume from the test volume during the procedure. A series of repetitive steps is then used to create different HCT levels in the test volume. A volume (x) is removed from the test volume, discarded, and replaced with an equivalent volume (x) of prepared saline solution. The system is allowed to stabilize, and the HCT is calculated at each stage based on the dilution of HCT. The average signal level of a "flat portion" of data at each HCT level tested is determined as shown, where $V_t$ is the total volume in the test set, $V_e$ is the volume exchanged (blood for saline), $H^0$ is the starting HCT (prior to volume exchange) and H' is the new HCT (post volume exchange). A hematocrit dependent curve is produced where the raw ratio is an input and the apparent hematocrit is the output.

Invasive, e.g., venous probes suitable for use in the present invention are known in the art. See, e.g., U.S. Patent Publication 2012/197136.

An Oral Probe for Non-invasive Monitoring of Fluorescence Intensities

An "optical conduit", as used in the present application, means a transparent optical waveguide, such as a fiber optic cable or an optically reflective pipe, which is capable of transmitting optical signals from one location to another.

An optical conduit may include an optical waveguide, such as a single fiber optic cable, or multiple optical waveguides arranged about a common optical source and optical interface, such as a bundle of fiber optic cables. An "optical interface" is the optical boundary that separates the terminal end of an optical conduit distal to an optical source or optical signal detector from an external environment.

A stabilized oral probe of the disclosure may be used to non-invasively monitor the fluorescent intensities of the fluorescent markers in the vascular system within the oral cavity of a mammalian subject. In an embodiment, the probe is placed under the tongue within the oral cavity.

The probe may include a longitudinal optical conduit having a proximal end and a distal end, wherein the distal end of the optical conduit forms a non-invasive interface between the optical conduit and the vascular system, and an oral stabilizing guide; wherein the fluorescent intensity of a fluorescent molecule in the vascular system is transmitted from the vascular system to the optical conduit through the optical interface at the distal end of the optical conduit to the proximal end of the optical conduit. The optical conduit may be a fiber optic cable. The proximal end of the optical conduit may be connected to a fluorescence detector to monitor the fluorescent intensities of the fluorescent markers in the vascular system. The optical conduit may transcend the oral stabilizing guide, or may be set in mechanical communication with the surface of the stabilizing guide such that the oral stabilizing guide limits the movement of the optical conduit. The oral stabilizing guide may include a dental inset. An oral stabilizing guide may also contain an optical guide protrusion for maintaining position of an optical conduit under the tongue.

The oral probe of the disclosure may further include a sterile sheath, which may include a uniform transparent material, or may include a transparent region and a moveable region. The oral probe may further include (a) a fitted region for maintaining a transparent sterile barrier between the optical interface and the tissue portion, and (b) a movable region for maintaining a sterile barrier between the optical positioning guide and the biological environment.

Light sources for exciting the fluorescent tags are known in the art. In the case of probes, they may be integral with the probe or separate from it.

## EXAMPLES

A table containing definitions of the variables used in the following examples 1-4 is set forth below.

Summary of Variables:

| Variable | Unit | Description |
|---|---|---|
| $x_1;x_2$ | mg | mg of VFI component 1; component 2 |
| $V_t$ | mL | Total blood volume |
| $V_s$ | mL | Volume of saline used for HCT calibration curve generation |
| $V_D$ | mL | Volume of VFI dose given |
| $H_{calib}$ | % | Percentage (decimal) of hematocrit known for whole blood used in calibration tests |
| $D_1; D_2$ | mg/mL | Concentration of component 1 or component 2 of VFI given in a dose |
| $S_1;S_2$ | --- | Signal level in generated calibration for Component 1; Component 2 |
| $m_1;m_2$ | $mg^{-1}$ | Slope of calibration curve for component 1; component 2 |
| $V_e$ | mL | Volume of blood-saline exchanged in hematocrit calibration |
| $H^o$ | % | Starting hematocrit prior to each volume exchange |
| $H'$ | % | Ending hematocrit after each volume exchange |
| $S_3;S_4$ | --- | Signal level in generated in HCT calibration for Component 1; Component 2 |
| $m_3; m_4$ | --- | Slope of Component 1 HCT calibration curve; Component 2 |
| $r$ | --- | Rate of attenuation of component 1 |
| $H$ | % | Hematocrit of the relevant sample |
| $b$ | --- | Constant intercept in component 2 hematocrit calibration curve |
| $R$ | --- | Raw ratio calculated from the hematocrit calibration curves for component 1 and 2 |

(continued)

| Variable | Unit | Description |
|---|---|---|
| K | --- | constant of the Ratio vs HCT calibration curve |
| q | --- | Rate of attenuation of the ratio of component 1 and 2 |
| $R_{To}$ | --- | Ratio of component 1 and component 2 signal levels at time zero; from a test |
| $S_{avg}$ | --- | Average stable component 2 signal calculated from a test |
| $H_{app}$ | % | Apparent hematocrit calculated from the raw ratio |
| $S_{calib}$ | --- | Signal level calculated from calibration curve and $H_{calib}$ |
| $S_{app}$ | --- | Signal level calculated from calibration curve and $H_{app}$ |
| c | --- | Correction factor applied to $S_{avg}$ to account for hematocrit difference |
| Sc | --- | Signal level after correction factor has been applied |
| $X_{eq}$ | mg | Calculated equivalent amount of material in a test to amount used in calibration |
| $V_{dist_{calib}}$ | mL | Volume of distribution of calibration tests |
| $X_{sub}$ | mg | Amount of a VFI component given to a test subject |
| $V_{dist_{sub}}$ | mL | Calculated volume of distribution of the test subject |
| $H_{sub}$ | % | Test subject's hematocrit calculated from the apparent HCT and a constant offset |
| $H_{os}$ | % | Constant offset between $H_{sub}$ and $H_{app}$ caused by fluid dynamics in the system |
| BV | mL | Blood volume of the test subject |

**Example 1:** Generation of calibration curves

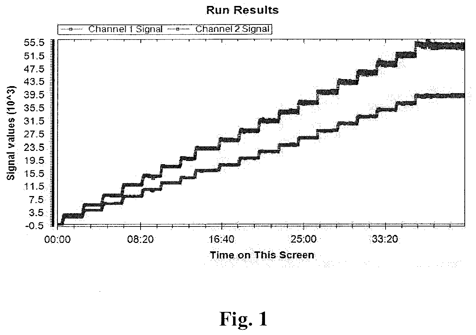

1. A step dose blood test set is run on a whole blood sample containing two fluorescent markers each having its distinct emission wavelength. An example of the results is shown in FIG. 1 with the upper curve representing the first emission signals from the first fluorescent marker or tag recorded in Channel 1 as the Channel 1 signal, and the second emission signals from the second fluorescent marker or tag recorded in Channel 2 as the Channel 2 signal. As discussed previously, this step dose blood test set can also be generated using one static marker having two fluorescent tags each tag having its distinct emission wavelength. Each fluorescent marker or each fluorescent tag may be referred to as a "fluorescent component" hereafter.
2. The average signal level of the "flat" or stable portion at each dose step for each fluorescent component is calculated.
3. Based on the known volume of blood ($V_t$) used, the known dose of VFI ($V_D$) and the known concentration of each VFI fluorescent component ($D_1$ or $D_2$) in the dose; the amount of each fluorescent marker present in the blood is calculated at each dose step (1).

$$x_{1;2} = V_D[D_{1;2}] \qquad (1)$$

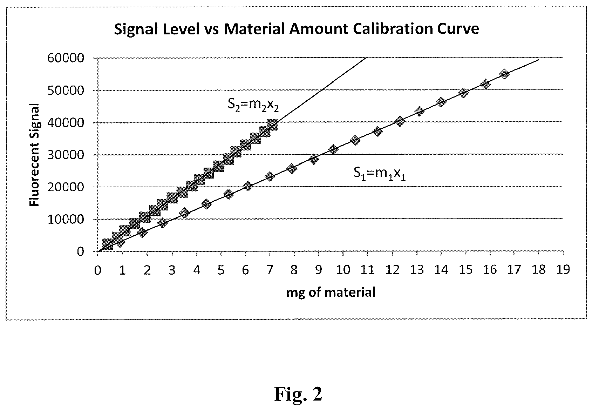

4. A fit line for the plot of each fluorescent component (intercept forced to zero) is generated, using the average signal level and amount of each component at each dose step calculated previously. The plot is shown in FIG. 2.

$$S_1 = m_1 x_1 \qquad (2)$$

$$S_2 = m_2 x_2 \qquad (3)$$

Where S is the signal level, m is the slope of the fit line and x is the amount (mg) of the material.

**Example 2:** Generation of a species specific hematocrit (HCT) calibration curve

1. A blood test is run with the single dose approach. With a known volume of blood ($V_t$) and a known HCT of the blood ($H_{calib}$), the volume of saline ($V_s$) needed for the test is calculated.

$$V_t - V_t H_{calib} = V_S \qquad (4)$$

2. The blood and the saline are equivalently dosed from the same VFI vial.

3. A predetermined volume of blood is removed from the test set and discarded. The same volume of dosed saline, as the blood previously removed, is injected back into the test set. This exchange will maintain the concentration of each component as well as the total volume of the test set, but alter the volume of distribution to HCT ratio. This step is repeated numerous times to generate multiple data points at which the volume of distribution and HCT ratio are different.

4. Each new point is allowed to stabilize before a new point is generated. A new HCT is calculated at each stable point.

$$\frac{(V_t - V_e)(H^0)}{V_t} = H' \qquad (5)$$

Where $V_t$ is the total volume in the apparatus, $V_e$ is the volume exchanged (blood for saline), $H^0$ is the starting HCT (prior to volume exchange), and $H'$ is the new HCT (post volume exchange).

5. The average signal level of a "flat" stable portion of data is taken at each HCT level generated during the test.

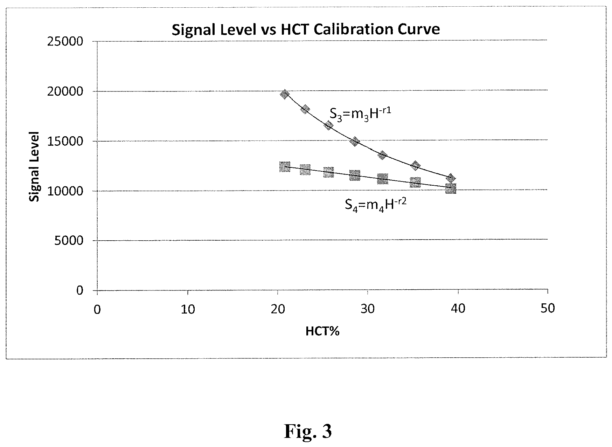

6. A plot of signal level vs. HCT is generated using the values calculated previously, as shown in FIG. 3.

7. A fit line is generated for each of the individual component plots. The equations generated are in the form:

$$S_3 = m_3 H^{-r1} \qquad (6)$$

$$S_4 = m_4 H^{-r2} \qquad (7)$$

Where S is the signal level, H is the HCT, m is the slope, and r is a rate.

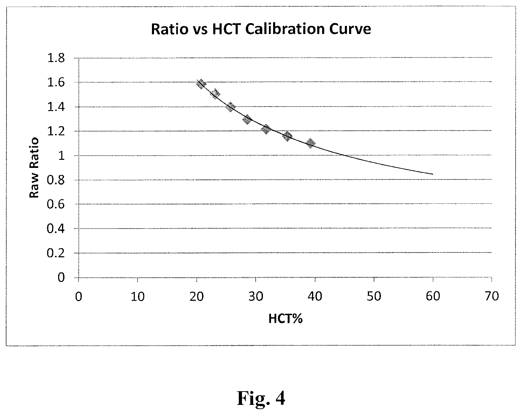

8. A fit line from the same HCT data taking the ratio of the signal levels of Component 1 to Component 2 (8), and plotting that ratio versus the HCT calculated at each stage in equation 5 is generated, as shown in FIG. 4.

$$R = S_3/S_4 \qquad (8)$$

The equation generated should take the form:

$$R = KH^{-q} \qquad (9)$$

Where R is the ratio, K is a slope, H is the HCT and q is a rate.

**Example 3:** Determining various biometric indicators

When a test is run on a subject, the "batch" of VFI must be known because the signal calibration and HCT calibration curves used for interpretation must be based on the same "batch" of VFI given to the subject.

1. From a test data sample of FIG. 5, the raw ratio at $T_0$ ($R_{T0}$) and the average stable Component 2 (FD003) signal level ($S_{avg}$) are extracted. The lower curve in FIG. 5 represents Channel 1 signals, and the upper curve represents Channel 2 signals.

2. Using the raw ratio at $T_0$ ($R_{T0}$), the apparent HCT of the subject is calculated from the Ratio vs HCT Calibration Curve.

$$R_{T0} = KH_{-q} \qquad (10)$$

$$H = H_{app} \qquad (11)$$

3. Using the calculated apparent HCT and the Signal Level vs. Material Amount Calibration Curve; the amount of correction, C, is calculated and applied to the average signal level component.
From Equation 7:

$$S_{calib} = m_4 H_{calib}^{-r} \qquad (12)$$

$$S_{app} = m_4 H_{app}^{-r} \qquad (13)$$

$$\textit{If } H_{app} < H_{calib} \textit{ then } S_{calib}/S_{app}$$

$$\textit{If } H_{app} > H_{calib} \textit{ then } S_{app}/S_{calib}$$

$$S_{calib}/S_{app} = C \qquad (14)$$

4. The correction factor, C, calculated in (14), is applied to the average signal level of component 2, $S_{avg}$, from the test data.

$$C * S_{avg} = S_C \qquad (15)$$

5. The corrected signal, Sc, is used in equation (16) to determine the equivalent amount of material of component 2 based on the Signal Level vs. Material Amount Calibration Curve.

$$S_C = m_2 x \qquad (16)$$

$$x = x_{eq} \qquad (17)$$

6. From a ratio of the known amount (mg) of VFI component 2 dosed in the subject, $x_{sub}$, to a known volume used in calibration, $V_{distcalib}$, and a calculated equivalent amount of component 2 (mg), $x_{eq}$, to a volume of subject's $V_{distcalib}$, the subject's volume of distribution is calculated.

$$V_{distcalib} = V_t - V_t H_{calib} \qquad (18)$$

$$x_{eq}/V_{distcalib} = x_{sub}/V_{distsub} \qquad (19)$$

$$V_{distsub} = x_{sub} V_{distcalib}/x_{eq} \qquad (20)$$

7. The subject's HCT from the apparent HCT and the HCT offset are calculated.

$$H_{sub} = H_{app} + H_{os} \qquad (21)$$

8. The blood volume from the volume of distribution of the subject and the calculated subject HCT is calculated.

$$BV = V_{distsub}/H_{sub} \qquad (22)$$

**Example 4:** Example Calculation

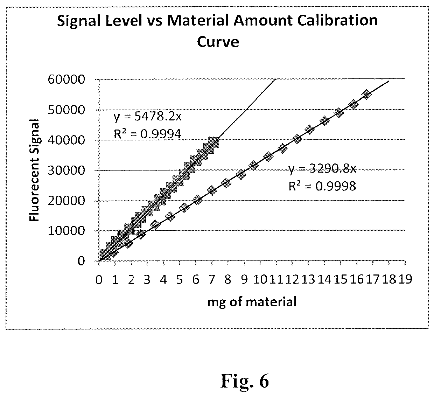

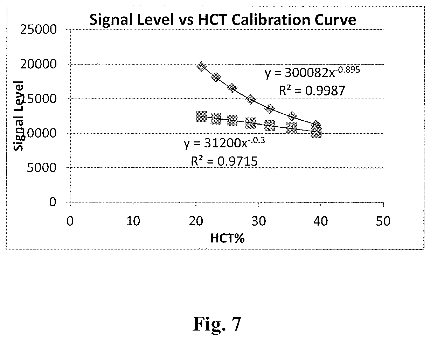

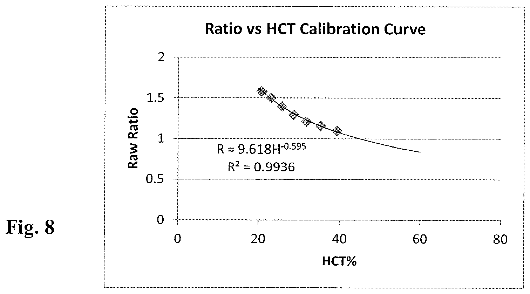

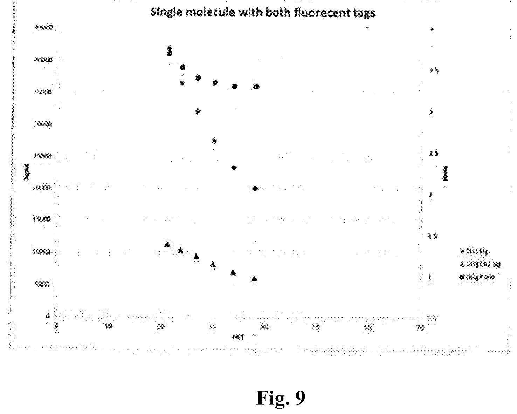

Calibration curves used in this example are shown in FIGS. 6 to 8. FIG. 9 is a calibration curve using one single static marker having two fluorescent tags.

For this example the following set of known parameters is used:

VFI dose concentration: 35mg/mL of Component 1 and 15mg/mL of Component 2
Dose Volume: 3.0mL
To Raw ratio: 1.2
Avg Stable Component 2 Signal Level: 12000
Calibration curve's test volume: 100mL
Calibration curve's test HCT: 38%.

1. From the raw ratio at $T_0$, $R_{T0}$, the apparent HCT of the subject is calculated from the Ratio vs HCT Calibration Curve.

$$1.2 = 9.618H^{-0.595} \qquad (23)$$

$$H_{app} = 33\% \qquad (24)$$

2. Using the calculated apparent HCT and the Signal Level vs HCT Calibration Curve; the amount of correction, C, needed to be applied to the average signal level of component 2 ($S_{avg}$), is calculated using the following (25, 26, 27).

$$S_{calib} = 31200(38)^{-0.3} \qquad (25)$$

$$S_{calib} = 10476$$

$$S_{app} = 31200(33)^{-0.3} \qquad (26)$$

$$S_{app} = 10930$$

$$H_{app} < H_{calib} \; so \; S_{calib}/S_{app}:$$

$$10476/10930 = 0.958 = C \qquad (27)$$

3. The correction factor, C, is applied to the average signal level of component 2, $S_{avg}$, from the test data.

$$(0.958)(12000) = S_C \qquad (28)$$

$$S_C = 11496$$

4. The corrected signal, Sc, in equation (16) is used to determine the equivalent amount of material of component 2 based on the Signal Level vs Material Amount Calibration Curve.

$$11496 = 5478.2x \qquad (29)$$

$$x = 2.09\text{mg} = x_{eq} \qquad (30)$$

5. From a ratio of the known amount (mg) of VFI component 2 dosed in the subject, $X_{sub}$, and a known volume used in calibration, $V_{distcalib}$, to a calculated equivalent amount of component 2 (mg), $x_{eq}$, and the volume of distribution of the subject, $V_{distcalib}$, the subject's volume of distribution is calculated.

$$V_{distcalib} = V_t - V_t H_{calib} \qquad (31)$$

$$V_{distcalib} = 100 - 100 * (.38) \qquad (32)$$

$$2.07/62 = (3 * 15)/V_{distsub} \qquad$$

$$V_{distsub} = 1334.9\text{mL} \qquad (33)$$

6. The subject's HCT is calculated from the apparent HCT and the HCT offset.

$$H_{sub} = 33 + 5 = 38\% \qquad (34)$$

7. Blood volume is calculated from the volume of distribution of the subject and the calculated subject HCT.

$$BV = 1334.9/0.38 \qquad (35)$$

$$BV = 3513\text{mL}$$

**Example 5:** Determining GFR In A Multi-Dose Context

The disclosed formula for multi-dose calculation addresses the plasma volume of any markers taken pre-dose (Blank). With such method, the total concentration of markers in the blood plasma is always calculated and used, since early and late decay rates are handled differently. With such technique, the case of a first test is treated the same as a follow on test, but setting the pre-dose blank values to zero.

The multi-dose formula and assumptions are as follows:

Blank = plasma concentration of any markers taken pre-dose
C1 = initial concentration of marker in plasma vs time
C2 = Concentration measured just before new follow on dose is given
C3 = Concentration vs time after follow on dose is given
$A_1$ = Initial magnitude of the fast decay rate
$B_1$ = Initial magnitude of the slow decay rate
$\alpha$ = fast decay rate
$\beta$ = slow decay rate
$t_1$ = time since initial dose of marker
$t_2$ = time since initial dose that follow on blank is drawn
$t_3$ = time since follow on dose is given.
mGFR = Calculated Glomerular Filtration Rate
Dose 1 = the initial dose

Dose 2 = the follow on dose
PV = Measured Plasma Volume at second dose

Assumes Blank = 0 which is the concentration measured pre-dose

$$C1 = A_1^{-\alpha t_1} + B_1^{-\beta t_1} \qquad (36)$$

New Blank taken just before follow on dose at time = $t_2$

$$\text{Assumes Blank is a measured value} \ = C2 \ = \ A_1^{-\alpha t_2} + B_1^{-\beta t_2} \qquad (37)$$

$$\text{New clearance} = C3 = \ A_2^{-\alpha t_3} + B_2^{-\beta t_3} \qquad (38)$$

Therefore:

$$mGFR = \frac{Dose\ 2}{\frac{A_2}{\alpha} + \frac{B_2 - C2}{\beta}} \ (39)$$

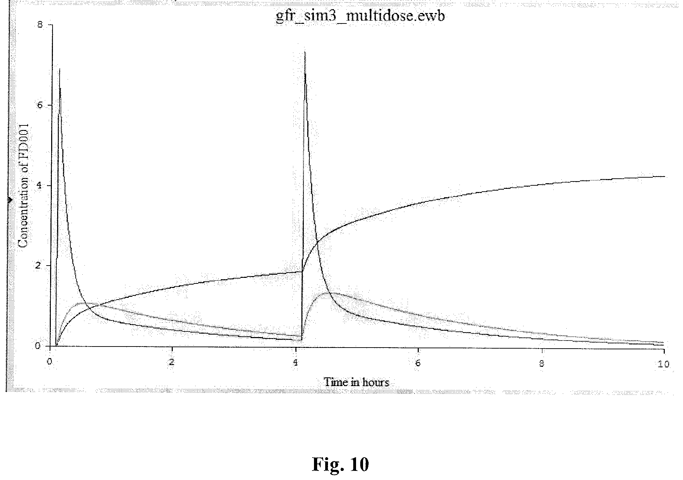

Where $A_2$ $B_2$ $\alpha$ and $\beta$ is the new clearance rate measured after the second dose. The symbols $\alpha$ and $\beta$ are defined above. $A_2$ and $B_2$ the initial magnitudes of the fast and slow decay rates of the markers in the second dose. This same equation can be used in any number of follow on doses.
Utilizing the equations and techniques set forth above, a method for determining a biometric indicator such as plasma concentration in a multi-dose context may be practiced.
The graphs illustrated in FIGs. 10-11 were generated from a computer simulation model and show how the fast and slow decay curves react during the follow on dose for both a normal and impaired patient. Figure 10 illustrates a normal mGFR showing a follow on dose of FD001. In Figure 10, signal 10A (Red) represents the plasma clearance of FD001 in units of ug/ml, while signal 12A (Green) represents the interstitial concentration of the FD001 in units of ug/ml, and signal 14A (Blue) represents the cumulative marker contained in the bladder during the testing time.

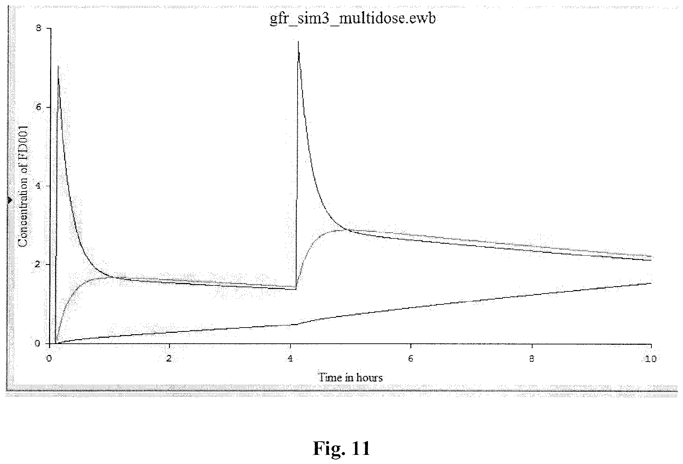

Figure 11 illustrates an impaired mGFR showing a follow on dose of FD001. In Figure11, signal 10B (Red) represents plasma clearance of FD001 in units of ug/ml, while signal 12BXX (Green) represents the interstitial concentration of the FD001 in units of ug/ml, and signal 14B (Blue) represents the cumulative marker contained in the bladder during the testing time. Note that the total kidney clearance remains proportional to total concentration of FD001, while the interstitial leakage is always relative to the new dose.
While the specific embodiments have been illustrated and described, numerous modifications come to mind, and the scope of protection is only limited by the scope of the accompanying claims.

**Claims**

**1.** An inj ectate for use in a method for measuring a biometric indicator of a mammalian subject, the inj ectate comprising

(i) a first fluorescent tag having a first excitation wavelength and a first emission wavelength;
(ii) a second fluorescent tag having a second excitation wavelength and a second emission wavelength, wherein both first and second fluorescent tags are conjugated to a static molecule, or wherein the first fluorescent tag is conjugated to the static molecule and the second fluorescent tag is conjugated to a dynamic molecule; and
(iii) an inj ectate carrier

wherein the method for measuring a biometric indicator comprises:

(a) calibrating an inj ectate to obtain a calibration identification of the inj ectate that contains parameters of the inj ectate,
(b) inputting the parameters of the calibration identification of the injectate to a fluorescent detector to calibrate the fluorescent detector;
(c) determining existing level of fluorescence in the mammalian subject;

(d) introducing the inj ectate into the vascular system of the mammalian subject;

(e) exciting the first fluorescent tag with a first excitation wavelength and exciting the second fluorescent marker with a second excitation wavelength at an optical interface of an optical probe and the vascular system, or in a sample taken from the vascular system;

(f) measuring the first emission intensity of the first emission wavelength of the first fluorescent tag and measuring the second intensity of the second emission wavelength of the second fluorescent tag at the optical interface of the optical probe and the vascular system, or in the sample taken from the vascular system, using the calibrated fluorescent detector, to obtain a spectrometric data set, which includes a first emission fluorescent intensity curve from the first fluorescent tag and a second emission fluorescent intensity curve from the second fluorescent tag;

(g) periodically repeating steps (c) through (f); and

(h) calculating the biometric indicator of the mammalian subject as a function of the first and second emission intensities measured in (f) and relative to the existing level of fluorescence measured in (c).

2. The injectate for use of claim 1, wherein the biometric indicator is plasma volume (PV), and step (h) of the method comprises: calculating the plasma volume of the mammalian subject as a function of the first and second emission intensities measured in (f) and relative to the existing level of fluorescence measured in (c).

3. The injectate for use of claim 2, wherein (e) is conducted 10 to 15 minutes after the introducing in (d), optionally wherein

   i) e-f and h are repeated at least once and wherein a change in plasma volume is calculated based on differences in two PV calculations, or

   ii) c-f and h are repeated at least once, and wherein a change in plasma volume is calculated based on differences in two PV calculations.

4. The injectate for use of claim 1, wherein the biometric indicator is glomerular filtration rate (GFR), and wherein steps (f)-(h) of the method comprise:

   (f) measuring the first emission intensity of the first emission wavelength of the first fluorescent tag and measuring the second intensity of the second emission wavelength of the second fluorescent tag at the optical interface of the optical probe and the vascular system, using the calibrated fluorescent detector, to obtain a spectrometric data set, which includes a first emission fluorescent intensity curve from the first fluorescent tag and a second emission fluorescent intensity curve from the second fluorescent tag, wherein the second fluorescent intensity curve further comprises peak fluorescent intensity of the second fluorescent tag conjugated to the dynamic marker which is extrapolated from amount of the second fluorescent tag contained in the injectate;

   (g) repeating steps (c) through (f) at least twice, wherein a first repetition is conducted at a time coinciding with or after equilibrium of the dynamic marker in plasma and interstitial fluid of the mammalian subject has been achieved; and

   (h) calculating the GFR of the mammalian subject as a function of the first and second emission intensities measured in (f) and (g) and relative to the existing level of fluorescence of (c).

5. The injectate for use of claim 1, wherein the biometric indicator is glomerular filtration rate (GFR), and wherein steps (f)-(h) of the method comprise:

   (f) measuring the first emission intensity of the first emission wavelength of the first fluorescent tag and measuring the second intensity of the second emission wavelength of the second fluorescent tag in a sample taken from the vascular system, using the calibrated fluorescent detector, to obtain a spectrometric data set, which includes a first emission fluorescent intensity curve from the first fluorescent tag and a second emission fluorescent intensity curve from the second fluorescent tag, wherein the second fluorescent intensity curve further comprises peak fluorescent intensity of the second fluorescent tag conjugated to the dynamic marker which is extrapolated from amount of the second fluorescent tag contained in the injectate and plasma volume, wherein plasma volume is determined as a function of the first and second emission intensities measured in (f) and relative to the existing level of fluorescence measured in (c);

   (g) repeating steps (c) through (f) at least twice, wherein a first repetition is conducted at a time coinciding with or after equilibrium of the dynamic marker in plasma and interstitial fluid of the mammalian subject has been achieved; and

   (h) calculating the GFR of the mammalian subject as a function of the first and second emission intensities measured in (f) and (g) and relative to the existing level of fluorescence of (c).

6. The injectate for use of claim 4 or 5, wherein (e) through (f) are repeated twice, optionally wherein i) first repetition of (e)-(f) is conducted 30 to 60 minutes after (d), or ii) second repetition of (e)-(f) is conducted 120 minutes after (d).

7. The injectate for use of claim 4 or 5, wherein (c)-(f) are repeated twice, and wherein repeated (c) is conducted 30 to 60 minutes after (c).

8. The injectate for use of claim 1, wherein the biometric indicator is hematocrit (HCT), and wherein the method steps (e)-(h) comprise:

   (e) exciting the first fluorescent tag with a first excitation wavelength and exciting the second fluorescent tag with a second excitation wavelength at an optical interface of an optical probe and the vascular system, optionally wherein (e) is conducted 10 to 15 minutes after the introducing in (d);
   (f) measuring the first emission intensity of the first emission wavelength of the first fluorescent tag and measuring the second intensity of the second emission wavelength of the second fluorescent tag at the optical interface of the optical probe and the vascular system, using the calibrated fluorescent detector, to obtain a spectrometric data set, which includes a first emission fluorescent intensity curve from the first fluorescent tag and a second emission fluorescent intensity curve from the second fluorescent tag, wherein the second fluorescent intensity curve further comprises peak fluorescent intensity of the second fluorescent tag conjugated to the dynamic molecule which is extrapolated from amount of the second fluorescent tag contained in the injectate;
   (g) periodically repeating steps (c) through (f); and
   (h) calculating the HCT of the mammalian subject as a function of a raw ratio of the first and second peak emission intensities measured in (f), and which is relative to the existing level of fluorescence measured in (c) and a species-specific HCT curve obtained prior to (h).

9. The injectate for use of of claim 1, wherein the biometric indicator is hematocrit (HCT), and wherein the method steps (e)-(h) comprise:

   (e) exciting the first fluorescent tag with a first excitation wavelength and exciting the second fluorescent tag with a second excitation wavelength at an optical interface of an optical probe and the vascular system, optionally wherein (e) is conducted 10 to 15 minutes after the introducing in (d);
   (f) measuring the first emission intensity of the first emission wavelength of the first fluorescent tag and measuring the second intensity of the second emission wavelength of the second fluorescent tag in a sample taken from the vascular system, using the calibrated fluorescent detector, to obtain a spectrometric data set, which includes a first emission fluorescent intensity curve from the first fluorescent tag and a second emission fluorescent intensity curve from the second fluorescent tag, wherein the second fluorescent intensity curve further comprises peak fluorescent intensity of the second fluorescent tag conjugated to the dynamic molecule which is extrapolated from amount of the second fluorescent tag contained in the injectate and plasma volume, wherein plasma volume is determined as a function of the first and second emission intensities measured in (f) and relative to the existing level of fluorescence measured in (c);
   (g) periodically repeating steps (c) through (f); and
   (h) calculating the HCT of the mammalian subject as a function of a raw ratio of the first and second peak emission intensities measured in (f), and which is relative to the existing level of fluorescence measured in (c) and a species-specific HCT curve obtained prior to (h).

10. The inj ectate for use of claim 1, wherein the biometric indicator is hematocrit (HCT), and wherein steps (a), (f)-(h) of the method comprise:

    (a) calibrating an inj ectate to obtain a calibration identification of the inj ectate that contains parameters of the inj ectate, wherein the inj ectate includes:

    (i) a first fluorescent tag having a first excitation wavelength and a first emission wavelength;
    (ii) a second fluorescent tag having a second excitation wavelength and a second emission wavelength, wherein the first fluorescent tag and the second fluorescent tag are each conjugated to a static molecule; and
    (iii) an inj ectate carrier;

    (f) measuring the first emission intensity of the first emission wavelength of the first fluorescent tag and measuring the second intensity of the second emission wavelength of the second fluorescent tag at the optical interface of the optical probe and the vascular system, or a sample taken from the vascular system, using the calibrated fluorescent detector, to obtain a spectrometric data set, which includes a first emission fluorescent intensity curve from the first fluorescent tag and a second emission fluorescent intensity curve from the second fluorescent tag;

(g) periodically repeating steps (c) through (f); and

(h) calculating the HCT of the mammalian subject as a function of a raw ratio of the first and second peak emission intensities measured in (f), and which is relative to the existing level of fluorescence measured in (c) and a species-specific HCT curve obtained prior to (h).

11. The injectate for use of any of claims 1-7, and claim 10, wherein;
(e) and (f) are conducted using a sample of whole blood obtained from the mammalian subject.

12. The injectate for use of any of claims 1-3, wherein;
the biometric indicator is PV, and the sample is plasma obtained from whole blood obtained from the mammalian subject.

13. The injectate for use of claim 7 or 8 wherein (e) is conducted 10 to 15 minutes after the introducing in (d) and (e) and (f) are conducted using a sample of whole blood obtained from the mammalian subject.

14. The injectate for use of any one of claims 1-10, wherein (e) and (f) are obtained using a probe, optionally wherein the probe is an oral probe or a venous probe.

15. The injectate for use of claim 8 or 10, wherein the probe is a venous probe, and wherein the raw ratio obtained in (h) is an apparent hematocrit, and wherein the method further comprises (i) determining a correction factor for the apparent hematocrit, and wherein said calculation in (h) comprises calculating the hematocrit of the mammalian subject based on the apparent hematocrit and the correction factor.

16. The injectate for use of any one of claims 1-15, wherein the static molecule is dextran having a molecular weight of 150 kDa.

17. The injectate for use of any one of claims 1-9 and 11-15, wherein the dynamic molecule is a dextran having a molecular weight from 5 to 7 kDa.

18. The injectate for use of any one of claims 1-17, wherein the first fluorescent tag is fluorescein or a derivative thereof optionally wherein the second fluorescent tag is rhodamine or a derivative thereof.

19. The injectate for use of any one of claims 1-3 and 11-18, wherein each of the first and second fluorescent tags is conjugated to the static molecule or wherein the first fluorescent tag is conjugated to the static molecule and the second fluorescent tag is conjugated to the dynamic molecule.

**Patentansprüche**

1. Injektat zur Verwendung in einem Verfahren zum Messen eines biometrischen Indikators eines Säugersubjekts, wobei das Injektat umfasst:

(i) ein erstes Fluoreszenz-Tag mit einer ersten Anregungswellenlänge und einer ersten Emissionswellenlänge;
(ii) ein zweites Fluoreszenz-Tag mit einer zweiten Anregungswellenlänge und einer zweiten Emissionswellenlänge, wobei sowohl das erste als auch das zweite Fluoreszenz-Tag an ein statisches Molekül konjugiert sind, oder wobei das erste Fluoreszenz-Tag an das statische Molekül konjugiert ist und das zweite Fluoreszenz-Tag an ein dynamisches Molekül konjugiert ist; und
(iii) einen Injektatträger,

wobei das Verfahren zum Messen eines biometrischen Indikators umfasst:

(a) Kalibrieren eines Injektats, um eine Kalibrierungsidentifikation des Injektats zu erhalten, die Parameter des Injektats enthält,
(b) Eingeben der Parameter der Kalibrierungsidentifikation des Injektats in einen Fluoreszenzdetektor, um den Fluoreszenzdetektor zu kalibrieren;

(c) Ermitteln eines bestehenden Niveaus der Fluoreszenz in dem Säugersubjekt;

(d) Einbringen des Injektats in das Gefäßsystem des Säugersubjekts;

(e) Anregen des ersten Fluoreszenz-Tags mit einer ersten Anregungswellenlänge und Anregen des zweiten Fluoreszenzmarkers mit einer zweiten Anregungswellenlänge an einer optischen Schnittstelle einer optischen Sonde und des Gefäßsystems, oder in einer Probe, die aus dem Gefäßsystem genommen wird;

(f) Messen der ersten Emissionsintensität der ersten Emissionswellenlänge des ersten Fluoreszenz-Tags und Messen der zweiten Intensität der zweiten Emissionswellenlänge des zweiten Fluoreszenz-Tags an der optischen Schnittstelle der optischen Sonde und des Gefäßsystems, oder in der Probe, die aus dem Gefäßsystem genommen wird, unter Verwendung des kalibrierten Fluoreszenzdetektors, um einen spektrometrischen Datensatz zu erhalten, der eine erste Emissionsfluoreszenzintensitätskurve aus dem ersten Fluoreszenz-Tag und eine zweite Emissionsfluoreszenzintensitätskurve aus dem zweiten Fluoreszenz-Tag einschließt;

(g) periodisches Wiederholen der Schritte (c) bis (f); und

(h) Berechnen des biometrischen Indikators des Säugersubjekts als Funktion der ersten und zweiten Emissionsintensitäten, die in (f) gemessen wurden, und relativ zu dem bestehenden Niveau der Fluoreszenz, das in (c) gemessen wurde.

2. Injektat zur Verwendung nach Anspruch 1, wobei der biometrische Indikator Plasmavolumen (PV) ist, und Schritt (h) des Verfahrens umfasst: Berechnen des Plasmavolumens des Säugersubjekts als Funktion der ersten und zweiten Emissionsintensitäten, die in (f) gemessen wurden, und relativ zu dem bestehenden Niveau der Fluoreszenz, das in (c) gemessen wurde.

3. Injektat zur Verwendung nach Anspruch 2, wobei (e) 10 bis 15 Minuten nach Einbringen in (d) durchgeführt wird, wobei gegebenenfalls

   i) e bis f und h mindestens ein Mal wiederholt werden, und wobei eine Änderung des Plasmavolumens basierend auf Differenzen der beiden PV-Berechnungen berechnet wird, oder

   ii) c bis f und h mindestens ein Mal wiederholt werden, und wobei eine Änderung des Plasmavolumens basierend auf Differenzen der beiden PV-Berechnungen berechnet wird.

4. Injektat zur Verwendung nach Anspruch 1, wobei der biometrische Indikator die glomeruläre Filtrationsrate (GFR) ist, und wobei die Verfahrensschritte (f) bis (h) umfassen:

   (f) Messen der ersten Emissionsintensität der ersten Emissionswellenlänge des ersten Fluoreszenz-Tags und Messen der zweiten Intensität der zweiten Emissionswellenlänge des zweiten Fluoreszenz-Tags an der optischen Schnittstelle der optischen Sonde und des Gefäßsystems unter Verwendung des kalibrierten Fluoreszenzdetektors, um einen spektrometrischen Datensatz, der eine erste Emissionsfluoreszenzintensitätskurve aus dem ersten Fluoreszenz-Tag und eine zweite Emissionsfluoreszenzintensitätskurve aus dem zweiten Fluoreszen-Tag einschließt, zu erhalten, wobei die zweite Fluoreszenzintensitätskurve des Weiteren den Spitzenwert der Fluoreszenzintensität des zweiten Fluoreszenz-Tags umfasst, das an den dynamischen Marker konjugiert ist, welcher aus der Menge des zweiten Fluoreszenz-Tags extrapoliert wird, die in dem Injektat enthalten ist;

   (g) Wiederholen der Schritte (c) bis (f) mindestens zwei Mal, wobei eine erste Wiederholung zu einer Zeit, die mit dem Erreichen des Gleichgewichts des dynamischen Markers in Plasma und interstitieller Flüssigkeit des Säugersubjekts zusammenfällt, oder danach durchgeführt wird; und

   (h) Berechnen der GFR des Säugersubjekts als Funktion der ersten und zweiten Emissionsintensitäten, die in (f) und (g) gemessen wurden, und relativ zu dem bestehenden Niveau der Fluoreszenz in (c).

5. Injektat zur Verwendung nach Anspruch 1, wobei der biometrische Indikator die glomeruläre Filtrationsrate (GFR) ist, und wobei die Verfahrensschritte (f) bis (h) umfassen:

   (f) Messen der ersten Emissionsintensität der ersten Emissionswellenlänge des ersten Fluoreszenz-Tags und Messen der zweiten Intensität der zweiten Emissionswellenlänge des zweiten Fluoreszenz-Tags in einer Probe, die aus dem Gefäßsystem genommen wurde, unter Verwendung des kalibrierten Fluoreszenzdetektors, um einen spektrometrischen Datensatz zu erhalten, der eine erste Emissionsfluoreszenzintensitätskurve aus dem ersten Fluoreszenz-Tag und eine zweite Emissionsfluoreszenzintensitätskurve aus dem zweiten Fluoreszenz-Tag einschließt, wobei die zweite Fluoreszenzintensitätskurve des Weiteren den Spitzenwert der Fluoreszenzintensität des zweiten Fluoreszenz-Tags umfasst, das an den dynamischen Marker konjugiert ist, welcher aus der Menge des zweiten Fluoreszenz-Tags extrapoliert wird, die in dem Injektat und Plasmavolumen enthalten ist, wobei das Plasmavolumen als Funktion der ersten und zweiten Emissionsintensitäten, die in (f) gemessen wurden, und relativ zu dem bestehenden Niveau der Fluoreszenz ermittelt wird, das in (c) gemessen wurde;

(g) Wiederholen der Schritte (c) bis (f) mindestens zwei Mal, wobei eine erste Wiederholung zu einer Zeit, die mit dem Erreichen des Gleichgewichts des dynamischen Markers in Plasma und interstitieller Flüssigkeit des Säugersubjekts zusammenfällt, oder danach durchgeführt wird; und

(h) Berechnen der GFR des Säugersubjekts als Funktion der ersten und zweiten Emissionsintensitäten, die in (f) und (g) gemessen wurden, und relativ zu dem bestehenden Niveau der Fluoreszenz in (c).

6. Injektat zur Verwendung nach Anspruch 4 oder 5, wobei (e) bis (f) zwei Mal wiederholt werden, wobei gegebenenfalls i) die erste Wiederholung von (e) bis (f) 30 bis 60 Minuten nach (d) durchgeführt wird, oder ii) die zweite Wiederholung von (e) bis (f) 120 Minuten nach (d) durchgeführt wird.

7. Injektat zur Verwendung nach Anspruch 4 oder 5, wobei (c) bis (f) zwei Mal wiederholt werden, und wobei die Wiederholung von (c) 30 bis 60 Minuten nach (c) durchgeführt wird.

8. Injektat zur Verwendung nach Anspruch 1, wobei der biometrische Indikator Hämatokrit (HCT) ist, und wobei die Verfahrensschritte (e) bis (h) umfassen:

(e) Anregen des ersten Fluoreszenz-Tags mit einer ersten Anregungswellenlänge und Anregen des zweiten Fluoreszenz-Tags mit einer zweiten Anregungswellenlänge an einer optischen Schnittstelle einer optischen Sonde und des Gefäßsystems, wobei gegebenenfalls (e) 10 bis 15 Minuten nach dem Einbringen in (d) durchgeführt wird;

(f) Messen der ersten Emissionsintensität der ersten Emissionswellenlänge des ersten Fluoreszenz-Tags und Messen der zweiten Intensität der zweiten Emissionswellenlänge des zweiten Fluoreszenz-Tags an der optischen Schnittstelle der optischen Sonde und des Gefäßsystems unter Verwendung des kalibrierten Fluoreszenzdetektors, um einen spektrometrischen Datensatz zu erhalten, der eine erste Emissionsfluoreszenzintensitätskurve aus dem ersten Fluoreszenz-Tag und eine zweite Emissionsfluoreszenzintensitätskurve aus dem zweiten Fluoreszenz-Tag einschließt, wobei die zweite Fluoreszenzintensitätskurve des Weiteren den Spitzenwert der Fluoreszenzintensität des zweiten Fluoreszenz-Tags umfasst, das an das dynamische Molekül konjugiert ist, welcher aus der Menge des zweiten Fluoreszenz-Tags extrapoliert wird, die in dem Injektat enthalten ist;

(g) periodisches Wiederholen der Schritte (c) bis (f); und

(h) Berechnen des HCTs des Säugersubjekts als Funktion eines rohen Verhältnisses der ersten und zweiten Spitzenwerte der Emissionsintensitäten, die in (f) gemessen wurden, und das relativ zu dem bestehenden Niveau der Fluoreszenz, das in (c) gemessen wurde, und einer speziesspezifischen HCT-Kurve ist, die vor (h) erhalten wurde.

9. Injektat zur Verwendung nach Anspruch 1, wobei der biometrische Indikator Hämatokrit (HCT) ist, und wobei die Verfahrensschritte (e) bis (h) umfassen:

(e) Anregen des ersten Fluoreszenz-Tags mit einer ersten Anregungswellenlänge und Anregen des zweiten Fluoreszenz-Tags mit einer zweiten Anregungswellenlänge an einer optischen Schnittstelle einer optischen Sonde und des Gefäßsystems, wobei gegebenenfalls (e) 10 bis 15 Minuten nach dem Einbringen in (d) durchgeführt wird;

(f) Messen der ersten Emissionsintensität der ersten Emissionswellenlänge des ersten Fluoreszenz-Tags und Messen der zweiten Intensität der zweiten Emissionswellenlänge des zweiten Fluoreszenz-Tags in einer Probe, die aus dem Gefäßsystem genommen wurde, unter Verwendung des kalibrierten Fluoreszenzdetektors, um einen spektrometrischen Datensatz zu erhalten, der eine erste Emissionsfluoreszenzintensitätskurve aus dem ersten Fluoreszenz-Tag und eine zweite Emissionsfluoreszenzintensitätskurve aus dem zweiten Fluoreszenz-Tag einschließt, wobei die zweite Fluoreszenzintensitätskurve des Weiteren den Spitzenwert der Fluoreszenzintensität des zweiten Fluoreszenz-Tags umfasst, das an das dynamische Molekül konjugiert ist, welcher aus der Menge des zweiten Fluoreszenz-Tags extrapoliert wird, die in dem Injektat und Plasmavolumen enthalten ist, wobei das Plasmavolumen als Funktion der ersten und zweiten Emissionsintensitäten, die in (f) gemessen wurden, und relativ zu dem bestehenden Niveau der Fluoreszenz ermittelt wird, das in (c) gemessen wurde;

(g) periodisches Wiederholen der Schritte (c) bis (f); und

(h) Berechnen des HCTs des Säugersubjekts als Funktion eines rohen Verhältnisses der ersten und zweiten Spitzenwerte der Emissionsintensitäten, die in (f) gemessen wurden, und das relativ zu dem bestehenden Niveau der Fluoreszenz, das in (c) gemessen wurde, und einer speziesspezifischen HCT-Kurve ist, die vor (h) erhalten wurde.

10. Injektat zur Verwendung nach Anspruch 1, wobei der biometrische Indikator Hämatokrit (HCT) ist, und wobei die Verfahrensschritte (a), (f) bis (h) umfassen:

(a) Kalibrieren eines Injektats, um eine Kalibrierungsidentifikation des Injektats zu erhalten, die Parameter des Injektats enthält, wobei das Injektat einschließt:

(i) ein erstes Fluoreszenz-Tag mit einer ersten Anregungswellenlänge und einer ersten Emissionswellenlänge;
(ii) ein zweites Fluoreszenz-Tag mit einer zweiten Anregungswellenlänge und einer zweiten Emissionswellenlänge, wobei das erste Fluoreszenz-Tag und das zweite Fluoreszenz-Tag jeweils an ein statisches Molekül konjugiert sind; und
(iii) einen Injektatträger;

(f) Messen der ersten Emissionsintensität der ersten Emissionswellenlänge des ersten Fluoreszenz-Tags und Messen der zweiten Intensität der zweiten Emissionswellenlänge des zweiten Fluoreszenz-Tags an der optischen Schnittstelle der optischen Sonde und des Gefäßsystems, oder einer Probe, die aus dem Gefäßsystem genommen wird, unter Verwendung des kalibrierten Fluoreszenzdetektors, um einen spektrometrischen Datensatz, der eine erste Emissionsfluoreszenzintensitätskurve aus dem ersten Fluoreszenz-Tag und eine zweite Emissionsfluoreszenzintensitätskurve aus dem zweiten Fluoreszenz-Tag einschließt, zu erhalten;
(g) periodisches Wiederholen der Schritte (c) bis (f); und
(h) Berechnen des HCTs des Säugersubjekts als Funktion eines rohen Verhältnisses der ersten und zweiten Spitzenwerte der Emissionsintensitäten, die in (f) gemessen wurden, und das relativ zu dem bestehenden Niveau der Fluoreszenz, das in (c) gemessen wurde, und einer speziesspezifischen HCT-Kurve ist, die vor (h) erhalten wurde.

11. Injektat zur Verwendung nach einem der Ansprüche 1 bis 7 und Anspruch 10, wobei:
(e) und (f) unter Verwendung einer Probe Vollblut durchgeführt werden, die von dem Säugersubjekt erhalten wurde.

12. Injektat zur Verwendung nach einem der Ansprüche 1 bis 3, wobei:
der biometrische Indikator PV ist und die Probe Plasma ist, das aus Vollblut erhalten wurde, welches von dem Säugersubjekt erhalten wurde.

13. Injektat zur Verwendung nach Anspruch 7 oder 8, wobei (e) 10 bis 15 Minuten nach dem Einbringen in (d) durchgeführt wird, und (e) und (f) unter Verwendung einer Probe Vollblut durchgeführt werden, die von dem Säugersubjekt erhalten wurde.

14. Injektat zur Verwendung nach einem der Ansprüche 1 bis 10, wobei (e) und (f) unter Verwendung einer Sonde erhalten werden, wobei die Sonde gegebenenfalls eine orale Sonde oder eine venöse Sonde ist.

15. Injektat zur Verwendung nach Anspruch 8 oder 10, wobei die Sonde eine venöse Sonde ist, und wobei das rohe Verhältnis, welches in (h) erhalten wird, ein scheinbarer Hämatokrit ist, und wobei das Verfahren des Weiteren (i) Ermitteln eines Korrekturfaktors für das scheinbare Hämatokrit umfasst, und wobei die Berechnung in (h) Berechnen des Hämatokrits des Säugersubjekts basierend auf dem scheinbaren Hämatokrit und dem Korrekturfaktor umfasst.

16. Injektat zur Verwendung nach einem der Ansprüche 1 bis 15, wobei das statische Molekül Dextran mit einem Molekulargewicht von 150 kDa ist.

17. Injektat zur Verwendung nach einem der Ansprüche 1 bis 9 und 11 bis 15, wobei das dynamische Molekül Dextran mit einem Molekulargewicht von 5 bis 7 kDa ist.

18. Injektat zur Verwendung nach einem der Ansprüche 1 bis 17, wobei das erste Fluoreszenz-Tag Fluoreszein oder ein Derivat davon ist, wobei gegebenenfalls das zweite Fluoreszenz-Tag Rhodamin oder ein Derivat davon ist.

19. Injektat zur Verwendung nach einem der Ansprüche 1 bis 3 und 11 bis 18, wobei jedes von dem ersten und dem zweiten Fluoreszenz-Tag an das statische Molekül konjugiert ist, oder wobei das erste Fluoreszenz-Tag an das statische Molekül konjugiert ist und das zweite Fluoreszenz-Tag an das dynamische Molekül konjugiert ist.

**Revendications**

1. Injectat destiné à être utilisé dans un procédé de mesure d'un indicateur biométrique d'un sujet mammifère, l'injectat comprenant

   (i) un premier marqueur fluorescent ayant une première longueur d'onde d'excitation et une première longueur d'onde d'émission ;
   (ii) un second marqueur fluorescent ayant une seconde longueur d'onde d'excitation et une seconde longueur d'onde d'émission, dans lequel à la fois les premier et second marqueurs fluorescents sont conjugués à une molécule statique, ou dans lequel le premier marqueur fluorescent est conjugué à la molécule statique et le second marqueur fluorescent est conjugué à une molécule dynamique ; et
   (iii) un excipient d'injectat

   dans lequel le procédé de mesure d'un indicateur biométrique comprend :

   (a) l'étalonnage d'un injectât pour obtenir une identification d'étalonnage de l'injectat qui contient des paramètres de l'injectat,
   (b) la saisie des paramètres de l'identification d'étalonnage de l'injectat dans un détecteur de fluorescence pour étalonner le détecteur de fluorescence ;
   (c) la détermination d'un niveau existant de fluorescence dans le sujet mammifère ;
   (d) l'introduction de l'injectat dans le système vasculaire du sujet mammifère ;
   (e) l'excitation du premier marqueur fluorescent avec une première longueur d'onde d'excitation et l'excitation du second marqueur fluorescent avec une seconde longueur d'onde d'excitation au niveau d'une interface optique d'une sonde optique et du système vasculaire, ou dans un échantillon prélevé dans le système vasculaire ;
   (f) la mesure de la première intensité d'émission de la première longueur d'onde d'émission du premier marqueur fluorescent et la mesure de la seconde intensité de la seconde longueur d'onde d'émission du second marqueur fluorescent au niveau de l'interface optique de la sonde optique et du système vasculaire, ou dans l'échantillon prélevé dans le système vasculaire, à l'aide du détecteur de fluorescence étalonné, de façon à obtenir un ensemble de données spectrométriques, qui comprend une première courbe d'intensité fluorescente d'émission en provenance du premier marqueur fluorescent et une seconde courbe d'intensité fluorescente d'émission en provenance du second marqueur fluorescent ;
   (g) la répétition périodique des étapes (c) à (f) ; et
   (h) le calcul de l'indicateur biométrique du sujet mammifère en fonction des première et seconde intensités d'émission mesurées en (f) et par rapport au niveau existant de fluorescence mesuré en (c).

2. Injectât destiné à être utilisé selon la revendication 1, dans lequel l'indicateur biométrique est le volume plasmatique (VP), et l'étape (h) du procédé comprend : le calcul du volume plasmatique du sujet mammifère en fonction des première et seconde intensités d'émission mesurées en (f) et par rapport au niveau de fluorescence existant mesuré en (c).

3. Injectât destiné à être utilisé selon la revendication 2, dans lequel l'étape (e) est menée 10 à 15 minutes après l'introduction dans (d), éventuellement dans lequel

   i) e, f et h sont répétées au moins une fois et dans lequel un changement de volume plasmatique est calculé sur la base de différentes dans deux calculs de VP, ou
   ii) c à f et h sont répétées au moins une fois, et dans lequel un changement de volume plasmatique est calculé sur la base de différentes dans deux calculs de VP.

4. Injectât destiné à être utilisé selon la revendication 1, dans lequel l'indicateur biométrique est le taux de filtration glomérulaire (TFG), et dans lequel les étapes (f) à (h) du procédé comprennent :

   (f) la mesure de la première intensité d'émission de la première longueur d'onde d'émission du premier marqueur fluorescent et la mesure de la seconde intensité de la seconde longueur d'onde d'émission du second marqueur fluorescent au niveau de l'interface optique de la sonde optique et du système vasculaire, à l'aide du détecteur de fluorescence étalonné, de façon à obtenir un ensemble de données spectrométriques, qui comprend une première courbe d'intensité fluorescente d'émission en provenance du premier marqueur fluorescent et une seconde courbe d'intensité fluorescente d'émission en provenance du second marqueur fluorescent, dans lequel la seconde courbe d'intensité fluorescente d'émission comprend en outre une intensité de fluorescence de pic du second marqueur fluorescent conjugué au marqueur dynamique qui est extrapolée à partir de la quantité du second marqueur fluorescent contenu dans l'injectat ;

(g) la répétition des étapes (c) à (f) au moins deux fois, une première répétition étant menée à un moment coïncidant avec un équilibre du marqueur dynamique dans le plasma et d'un fluide interstitiel du sujet mammifère, ou après que celui-ci a été atteint ; et

(h) le calcul du TFG du sujet mammifère en fonction des première et seconde intensités d'émission mesurées en (f) et (g) par rapport au niveau existant de fluorescence de (c) .

5. Injectât destiné à être utilisé selon la revendication 1, dans lequel l'indicateur biométrique est le taux de filtration glomérulaire (TFG), et dans lequel les étapes (f) à (h) du procédé comprennent :

(f) la mesure de la première intensité d'émission de la première longueur d'onde d'émission du premier marqueur fluorescent et la mesure de la seconde intensité de la seconde longueur d'onde d'émission du second marqueur fluorescent dans un échantillon prélevé dans le système vasculaire, à l'aide du détecteur de fluorescence étalonné, de façon à obtenir un ensemble de données spectrométriques, qui comprend une première courbe d'intensité fluorescente d'émission en provenance du premier marqueur fluorescent et une seconde courbe d'intensité fluorescente d'émission en provenance du second marqueur fluorescent, dans lequel la seconde courbe d'intensité fluorescente d'émission comprend en outre une intensité de fluorescence de pic du second marqueur fluorescent conjugué au marqueur dynamique qui est extrapolée à partir de la quantité du second marqueur fluorescent contenu dans l'injectat et le volume plasmatique, dans lequel le volume plasmatique est déterminé en fonction des première et seconde intensités d'émission mesurées en (f) et par rapport au niveau de fluorescence existant mesuré en (c) ;

(g) la répétition des étapes (c) à (f) au moins deux fois, une première répétition étant menée à un moment coïncidant avec un équilibre du marqueur dynamique dans le plasma et d'un fluide interstitiel du sujet mammifère, ou après que celui-ci a été atteint ; et

(h) le calcul du TFG du sujet mammifère en fonction des première et seconde intensités d'émission mesurées en (f) et (g) et par rapport au niveau existant de fluorescence de (c).

6. Injectât destiné à être utilisé selon la revendication 4 ou 5, dans lequel les étapes (e) et (f) sont répétées deux fois, éventuellement dans lequel i) une première répétition des étapes (e) et (f) est menée 30 à 60 minutes après (d), ou ii) une seconde répétition des étapes (e) et (f) est menée 120 minutes après (d).

7. Injectât destiné à être utilisé selon la revendication 4 ou 5, dans lequel les étapes (c) à (f) sont répétées deux fois, et dans lequel la répétition de l'étape (c) est menée 30 à 60 minutes après (c).

8. Injectât destiné à être utilisé selon la revendication 1, dans lequel l'indicateur biométrique est l'hématocrite (HCT), et dans lequel les étapes de procédé (e) à (h) comprennent :

(e) l'excitation du premier marqueur fluorescent avec une première longueur d'onde d'excitation et l'excitation du second marqueur fluorescent avec une seconde longueur d'onde d'excitation au niveau d'une interface optique d'une sonde optique et du système vasculaire, éventuellement dans lequel l'étape (e) est menée 10 à 15 minutes après l'introduction dans (d) ;

(f) la mesure de la première intensité d'émission de la première longueur d'onde d'émission du premier marqueur fluorescent et la mesure de la seconde intensité de la seconde longueur d'onde d'émission du second marqueur fluorescent au niveau de l'interface optique de la sonde optique et du système vasculaire, à l'aide du détecteur de fluorescence étalonné, de façon à obtenir un ensemble de données spectrométriques, qui comprend une première courbe d'intensité fluorescente d'émission en provenance du premier marqueur fluorescent et une seconde courbe d'intensité fluorescente d'émission en provenance du second marqueur fluorescent, dans lequel la seconde courbe d'intensité fluorescente d'émission comprend en outre une intensité de fluorescence de pic du second marqueur fluorescent conjugué au marqueur dynamique qui est extrapolée à partir de la quantité du second marqueur fluorescent contenu dans l'injectat ;

(g) la répétition périodique des étapes (c) à (f) ; et

(h) le calcul du HCT du sujet mammifère en fonction d'un rapport brut des première et seconde intensités d'émission de pic mesurées en (f), et qui est relatif au niveau de fluorescence existant mesuré en (c) et à une courbe HCT spécifique à l'espèce obtenue avant (h).

9. Injectât destiné à être utilisé selon la revendication 1, dans lequel l'indicateur biométrique est l'hématocrite (HCT), et dans lequel les étapes de procédé (e) à (h) comprennent :

(e) l'excitation du premier marqueur fluorescent avec une première longueur d'onde d'excitation et l'excitation du second marqueur fluorescent avec une seconde longueur d'onde d'excitation au niveau d'une interface optique d'une sonde optique et du système vasculaire, éventuellement dans lequel l'étape (e) est menée 10 à 15 minutes après l'introduction dans (d) ;
(f) la mesure de la première intensité d'émission de la première longueur d'onde d'émission du premier marqueur fluorescent et la mesure de la seconde intensité de la seconde longueur d'onde d'émission du second marqueur fluorescent dans un échantillon prélevé dans le système vasculaire, à l'aide du détecteur de fluorescence étalonné, de façon à obtenir un ensemble de données spectrométriques, qui comprend une première courbe d'intensité fluorescente d'émission en provenance du premier marqueur fluorescent et une seconde courbe d'intensité fluorescente d'émission en provenance du second marqueur fluorescent, dans lequel la seconde courbe d'intensité fluorescente d'émission comprend en outre une intensité de fluorescence de pic du second marqueur fluorescent conjugué au marqueur dynamique qui est extrapolée à partir de la quantité du second marqueur fluorescent contenu dans l'injectat et le volume plasmatique, dans lequel le volume plasmatique est déterminé en fonction des première et seconde intensités d'émission mesurées en (f) et par rapport au niveau de fluorescence existant mesuré en (c) ;
(g) la répétition périodique des étapes (c) à (f) ; et
(h) le calcul du HCT du sujet mammifère en fonction d'un rapport brut des première et seconde intensités d'émission de pic mesurées en (f), et qui est relatif au niveau de fluorescence existant mesuré en (c) et à une courbe HCT spécifique à l'espèce obtenue avant (h).

10. Injectat destiné à être utilisé selon la revendication 1, dans lequel l'indicateur biométrique est l'hématocrite (HCT), et dans lequel les étapes (a), (f) à (h) du procédé comprennent :

(a) l'étalonnage d'un injectât pour obtenir une identification d'étalonnage de l'injectat qui contient des paramètres de l'injectat, dans lequel l'injectat comprend :

(i) un premier marqueur fluorescent ayant une première longueur d'onde d'excitation et une première longueur d'onde d'émission ;
(ii) un second marqueur fluorescent ayant une seconde longueur d'onde d'excitation et une seconde longueur d'onde d'émission, dans lequel le premier marqueur fluorescent et le second marqueur fluorescent sont chacun conjugués à une molécule statique ; et
(iii) un excipient d'injectat ;

(f) la mesure de la première intensité d'émission de la première longueur d'onde d'émission du premier marqueur fluorescent et la mesure de la seconde intensité de la seconde longueur d'onde d'émission du second marqueur fluorescent au niveau de l'interface optique de la sonde optique et du système vasculaire, ou un échantillon prélevé dans le système vasculaire, à l'aide du détecteur de fluorescence étalonné, de façon à obtenir un ensemble de données spectrométriques, qui comprend une première courbe d'intensité fluorescente d'émission en provenance du premier marqueur fluorescent et une seconde courbe d'intensité fluorescente d'émission en provenance du second marqueur fluorescent ;
(g) la répétition périodique des étapes (c) à (f) ; et
(h) le calcul du HCT du sujet mammifère en fonction d'un rapport brut des première et seconde intensités d'émission de pic mesurées en (f), et qui est relatif au au niveau de fluorescence existant mesuré en (c) et à une courbe HCT spécifique à l'espèce obtenue avant (h).

11. Injectat destiné à être utilisé selon l'une quelconque des revendications 1 à 7 et 10, dans lequel : les étapes (e) et (f) sont menées en utilisant un échantillon de sang total obtenu du sujet mammifère.

12. Injectât destiné à être utilisé selon l'une quelconque des revendications 1 à 3, dans lequel : l'indicateur biométrique est le VP, et l'échantillon est du plasma obtenu du sang total obtenu du sujet mammifère.

13. Injectat destiné à être utilisé selon la revendication 7 ou 8 dans lequel l'étape (e) est menée 10 à 15 minutes après l'introduction dans (d) et les étapes (e) et (f) sont menées en utilisant un échantillon de sang total obtenu du sujet mammifère.

14. Injectat destiné à être utilisé selon l'une quelconque des revendications 1 à 10, dans lequel les étapes (e) et (f) sont obtenues à l'aide d'une sonde, éventuellement dans lequel la sonde est une sonde orale ou une sonde veineuse.

15. Injectat destiné à être utilisé selon la revendication 8 ou 10, dans lequel la sonde est une sonde veineuse, et dans lequel le rapport brut obtenu en (h) est un hématocrite apparent, et dans lequel le procédé comprend en outre (i) la détermination d'un facteur de correction pour l'hématocrite apparent, et dans lequel ledit calcul dans (h) comprend le calcul de l'hématocrite du sujet mammifère sur la base de l'hématocrite apparent et du facteur de correction.

16. Injectat destiné à être utilisé selon l'une quelconque des revendications 1 à 15, dans lequel la molécule statique est le dextrane ayant un poids moléculaire de 150 kDa.

17. Injectat destiné à être utilisé selon l'une quelconque des revendications 1 à 9 et 11 à 15, dans lequel la molécule dynamique est le dextrane ayant un poids moléculaire de 5 à 7 kDa.

18. Injectat destiné à être utilisé selon l'une quelconque des revendications 1 à 17, dans lequel le premier marqueur fluorescent est la fluorescéine ou un dérivé de celle-ci, éventuellement dans lequel le second marqueur fluorescent est la rhodamine ou un dérivé de celle-ci.

19. Injectat destiné à être utilisé selon l'une quelconque des revendications 1 à 3 et 11 à 18, dans lequel chacun des premier et second marqueurs fluorescents est conjugué à la molécule statique ou dans lequel le premier marqueur fluorescent est conjugué à la molécule statique et le second marqueur fluorescent est conjugué à la molécule dynamique.

**Fig. 1**

**Fig. 2**

**Signal Level vs HCT Calibration Curve**

$S_3 = m_3 H^{-r1}$

$S_4 = m_4 H^{-r2}$

Signal Level

HCT%

**Fig. 3**

**Fig. 4**

**Fig. 5**

**Fig. 6**

**Fig. 7**

Fig. 8

Ratio vs HCT Calibration Curve

$R = 9.618H^{-0.595}$

$R^2 = 0.9936$

Fig. 9

**Fig. 10**

**Fig. 11**

**REFERENCES CITED IN THE DESCRIPTION**

**Patent documents cited in the description**

- US 425827
- US 12946471 B
- US 2009040994 W
- US 201032997 W
- EP 2814393 A
- US 2012197136 A

**Non-patent literature cited in the description**

- **WANG et al.** A portable fiberoptic ratiometric fluorescence analyzer provides rapid point-of-care determination of glomerular filtration rate in large animals. *Kidney International,* 2012